(19) Europäisches Patentamt
European Patent Office
Office européen des brevets

(11) Veröffentlichungsnummer: **0 098 448**
**A2**

(12) # EUROPÄISCHE PATENTANMELDUNG

(21) Anmeldenummer: **83106026.4**

(22) Anmeldetag: **21.06.83**

(51) Int. Cl.³: **C 07 D 235/18**, C 07 D 471/04, C 07 D 473/00, A 61 K 31/415, A 61 K 31/435, A 61 K 31/52 // (C07D471/04, 235/00, 221/00)

(30) Priorität: **01.07.82 DE 3224512**

(43) Veröffentlichungstag der Anmeldung: **18.01.84 Patentblatt 84/3**

(84) Benannte Vertragsstaaten: **AT BE CH DE FR IT LI LU NL SE**

(71) Anmelder: **Dr. Karl Thomae GmbH, Postfach 1755, D-7950 Biberach (Riss) (DE)**

(72) Erfinder: **Hauel, Norbert, Dr. Dipl.-Chem., Händelstrasse 12, D-7950 Biberach 1 (DE)**
Erfinder: **Austel, Volkhard, Dr. Dipl.-Chem., Kapellenweg 7, D-7950 Biberach 1 (DE)**
Erfinder: **Heider, Joachim, Dr. Dipl.-Chem., Am Hang 3, D-7951 Warthausen 1 (DE)**
Erfinder: **Reiffen, Manfred, Dr. Dipl.-Chem., Amriswilstrasse 7, D-7950 Biberach 1 (DE)**
Erfinder: **Diederen, Willi, Dr., Haldenstrasse 1a, D-7980 Biberach 1 (DE)**

(54) **Neue Imidazolderivate, ihre Herstellung und diese Verbindungen enthaltende Arzneimittel.**

(57) Neue Imidazolderivate der allgemeinen Formel

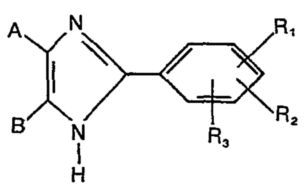

,(I)

in der
A und B zusammen mit den beiden dazwischenliegenden Kohlenstoffatomen eine Gruppe der Formel

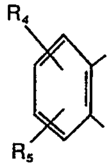 , 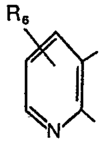 , 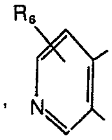 oder 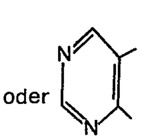 ,

wobei der
Phenyl- und Pyridinring gegebenenfalls substituiert sein kann,

$R_1$ eine Alkansulfonyloxy-, Trifluormethansulfonyloxy-, Alkansulfonylamino-, Trifluormethansulfonylamino-, N-Alkylalkansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Nitro- oder Cyangruppe,

$R_2$ eine Alkyl-, Alkoxy- oder Dialkylaminogruppe und

$R_3$ ein Wasserstoffatom oder eine Alkoxygruppe bedeuten, deren Tautomere und deren Säureadditionssalze, insbesondere deren physiologisch verträgliche Säureadditionssalze mit anorganischen oder organischen Säuren, welche wertvolle pharmakologische Eigenschaften aufweisen, insbesondere eine Wirkung auf die Kontraktilität des Herzens.

Die neuen Verbindungen können nach für analoge Verbindungen üblichen Verfahren hergestellt werden.

EP 0 098 448 A2

DR. KARL THOMAE GMBH
D-7950 Biberach 1

Case 5/865
Dr.Fl/pf

# Neue Imidazolderivate, ihre Herstellung und diese Verbindungen enthaltende Arzneimittel

In der US-A-3.985.891, EP-A-0.024.290 und EP-A-0.022.495 werden bereits Imidazolderivate beschrieben, welche wertvolle pharmakologische Eigenschaften aufweisen.

Es wurde nun gefunden, daß die neuen Imidazolderivate der allgemeinen Formel

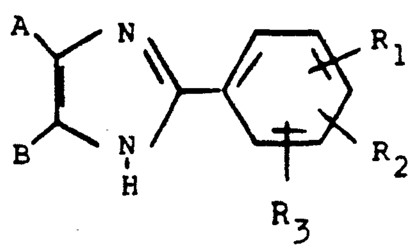

,(I)

deren Tautomere und deren Säureadditonssalze, insbesondere deren physiologisch verträgliche Säureadditionssalze mit anorganischen oder organischen Säuren, welche sich von den bereits bekannten Imidazolderivaten durch den Substituenten $R_1$ unterscheiden, überlegene pharmakologische Eigenschaften aufweisen, insbesondere eine Wirkung auf die Kontraktilität des Herzmuskels.

In der obigen allgemeinen Formel I bedeutet

A und B zusammen mit den beiden dazwischenliegenden Kohlenstoffatomen eine Gruppe der Formel

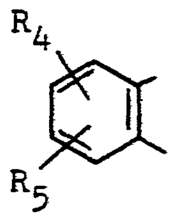 , 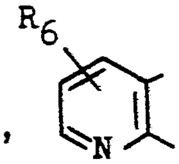 , 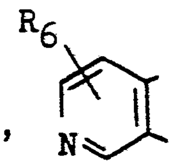 oder 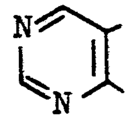 ,wobei

$R_4$ ein Wasserstoffatom- oder Halogenatom, eine Alkyl-, Hydroxy-, Alkoxy-, Trifluormethyl-, Cyano-, Carboxy-, Alkoxycarbonyl-, Aminocarbonyl-, Alkylaminocarbonyl-, Dialkylaminocarbonyl-, Hydroxyalkyl-, Nitro-, Amino-, Alkanoylamino-, Alkoxycarbonylamino-, Aminocarbonylamino-, Alkylaminocarbonylamino-, Dialkylaminocarbonylamino-, Alkansulfonylamino- oder N-Alkyl-alkansulfonylaminogruppe,

$R_5$ ein Wasserstoffatom- oder Halogenatom, eine Alkyl-, Hydroxy- oder Alkoxygruppe und

$R_6$ ein Wasserstoff- oder Halogenatom oder eine Alkylgruppe darstellen, wobei die vorstehend erwähnten Alkylteile jeweils 1 bis 3 Kohlenstoffatome enthalten können,

$R_1$ eine Alkansulfonyloxy-, Trifluormethansulfonyloxy-, Alkansulfonylamino-, N-Alkyl-alkansulfonylamino-, Trifluormethansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, wobei der Alkylteil der vorstehend genannten Gruppen jeweils 1 bis 3 Kohlenstoffatome enthalten kann, eine durch eine Amino-, Alkylamino-, Dialkylamino- oder cyclische Iminogruppe substituierte Sulfonylgruppe, wobei der Alkylteil jeweils 1 bis 5 Kohlenstoffatome und die Iminogruppe 4 bis 7 Kohlenstoffatome enthalten und gleichzeitig eine Methylengruppe in 4-Stellung der cyclischen Iminogruppe durch ein Schwefel- oder Sauerstoffatom ersetzt sein kann, eine Nitro- oder Cyangruppe,

$R_2$ eine Alkyl-, Alkoxy- oder Dialkylaminogruppe mit jeweils 1 bis 3 Kohlenstoffatomen in jedem Alkylteil und

$R_3$ ein Wasserstoffatom oder eine Alkoxygruppe mit 1 bis 3 Kohlenstoffatomen.

Gegenstand der vorliegenden Erfindung sind somit die neuen Benzimidazole, Imidazo[4,5-b]pyridine, Imidazo[4,5-c]pyridine sowie Purine der obigen allgemeinen Formel I, deren Tautomere, deren Säureadditionssalze, insbesondere deren physiologisch verträgliche Säureadditionssalze mit anorganischen oder organischen Säuren, und Verfahren zu ihrer Herstellung sowie diese Verbindungen enthaltende Arzneimittel.

Für die bei der Definition der Reste eingangs erwähnten Bedeutungen kommt beispielsweise

Für $R_1$ die Bedeutung der Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, Isopropylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N-n-Propyl-n-propansulfonylamino-, N-Methyl-trifluormethansulfonylamino-, N-Ethyl-trifluormethansulfonylamino-, N-Isopropyl-trifluormethansulfonylamino-, Nitro-, Cyan-, Carboxy-, Methoxycarbonyl-, Ethoxycarbonyl-, Propoxycarbonyl-, Isopropoxycarbonyl-, Aminocarbonyl-, Methylaminocarbonyl-, Ethylaminocarbonyl-, Dimethylaminocarbonyl-, Di-n-propylaminocarbonyl-, N-Methyl-ethylaminocarbonyl-, Pyrrolidinosulfonyl-, Piperidinosulfonyl-, Hexamethyleniminosulfonyl-, Aminosulfonyl-, Methylaminosulfonyl-, Ethylaminosuflonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methyl-isopropylaminosulfonyl- oder Morpholinosulfonylgruppe,

für $R_2$ die der Methyl-, Ethyl-, Propyl-, Isopropyl-, Methoxy-, Ethoxy-, Propoxy-, Dimethylamino-, Diethylamino- oder N-Methyl-n-propylaminogruppe,

für $R_3$ die des Wasserstoffatoms, der Methoxy-, Ethoxy-, Propoxy- oder Isopropoxygruppe,

für $R_4$ die des Wasserstoff-, Fluor-, Chlor- oder Bromatoms, der Trifluormethyl-, Methyl-, Ethyl-, Propyl-, Isopropyl-, Hydroxy-, Methoxy-, Ethoxy-, Propoxy-, Isopropoxy-, Cyano-, Carboxy-, Methoxycarbonyl-, Ethoxycarbonyl-, Isopropoxycarbonyl-, Aminocarbonyl-, Methylaminocarbonyl-, Ethylaminocarbonyl-, Propylaminocarbonyl-, Dimethylaminocarbonyl-, Diethylaminocarbonyl-, Dipropylaminocarbonyl-, Methylethylaminocarbonyl-, Methyl-isopropylaminocarbonyl-, Ethylpropylaminocarbonyl-, Hydroxymethyl-, 1-Hydroxyethyl-, 2-Hydroxyethyl-, 1-Hydroxypropyl-, 2-Hydroxypropyl-, 3-Hydroxypropyl-, Nitro-, Amino-, Formylamino-, Acetamino-, Propionylamino-, Methoxycarbonylamino-, Ethoxycarbonylamino-, Propoxycarbonylamino-, Isopropoxycarbonylamino-, Aminocarbonylamino-, Methylaminocarbonylamino-, Ethylaminocarbonylamino-, Propylaminocarbonylamino-, Dimethylaminocarbonylamino-, Diethylaminocarbonylamino-, Diisopropylaminocarbonylamino-, Methyl-ethylaminocarbonylamino-, Ethylpropylaminocarbonylamino-, Methansulfonylamino-, Ethansulfonylamino-, Propansulfonylamino-, Isopropansulfonylamino-, N-Methyl-methansulfonylamino-, N-Ethyl-methansulfonylamino-, N-Propylmethansulfonylamino- oder N-Ethyl-ethansulfonylaminogruppe,

für $R_5$ die des Wasserstoff-, Fluor-, Chlor- oder Bromatoms, der Methyl-, Ethyl-, Propyl-, Isopropyl-, Hydroxy-,

Methoxy-, Ethoxy-, Propoxy- oder Isopropoxygruppe und

für $R_6$ die des Wasserstoff-, Fluor-, Chlor- oder Bromatoms, der Methyl-, Ethyl-, Propyl- oder Isopropylgruppe in Betracht.

Bevorzugte Verbindungen der obigen allgemeinen Formel I sind diejenigen, in denen

A und B zusammen mit den beiden dazwischenliegenden Kohlenstoffatomen eine Gruppe der Formel

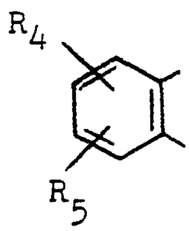 , 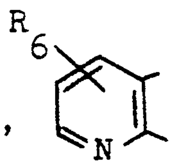 , 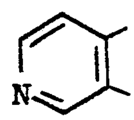 oder 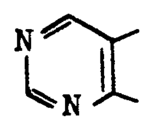 ,wobei

$R_4$ ein Wasserstoff-, Fluor-, Chlor- oder Bromatom, eine Trifluormethyl-, Cyan-, Methyl-, Hydroxy-, Methoxy-, Hydroxymethyl-, Carboxy-, Methoxycarbonyl-, Aminocarbonyl-, Methylaminocarbonyl-, Nitro-, Amino-, Acetylamino-, Methoxycarbonylamino-, Methansulfonylamino-, Aminocarbonylamino- oder Methylaminocarbonylaminogruppe,

$R_5$ ein Wasserstoffatom, eine Methyl- oder Methoxygruppe und

$R_6$ eine Methylgruppe, ein Wasserstoff- oder Chloratom darstellen,

$R_1$ eine Alkansulfonyloxy-, Trifluormethansulfonyloxy-, Alkylsulfenylmethyl-, Alkylsulfinylmethyl-, Alkylsulfonylmethyl-, Alkansulfonylamino-, N-Alkyl-alkansulfonylamino-, Trifluormethansulfonylamino- oder N-Alkyl-trifluormethansulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2

Kohlenstoffatome enthalten kann, eine Nitro-, Cyan- oder Alkylaminosulfonylgruppe mit 1 bis 4 Kohlenstoffatomen,

$R_2$ eine Alkylgruppe mit 1 bis 3 Kohlenstoffatomen, eine Alkoxy- oder Dialkylaminogruppe mit 1 oder 2 Kohlenstoffatomen in jedem Alkylteil und

$R_3$ ein Wasserstoffatom oder die Methoxygruppe bedeuten, deren Tautomeren und deren Säureadditionssalze, insbesondere deren physiologisch verträgliche Säureadditionssalze mit anorganischen oder organischen Säuren.

Besonders bevorzugte Verbindungen sind jedoch die Verbindungen der allgemeinen Formel

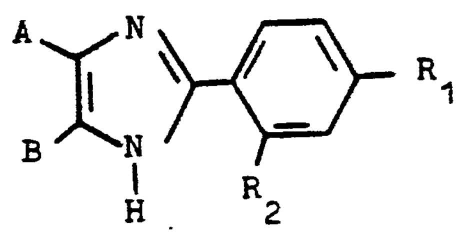 ,(Ia)

in der

A und B zusammen mit den beiden dazwischenliegenden Kohlenstoffatomen eine Gruppe der Formel

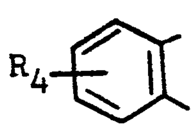 , 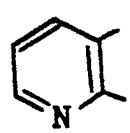 , 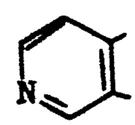 oder 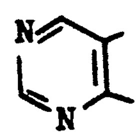 ,wobei

$R_4$ ein Wasserstoff- oder Fluoratom, eine Methyl-, Hydroxy-, Methoxycarbonyl-, Aminocarbonyl-, Amino-, Acetylamino- oder Cyangruppe darstellt,

$R_1$ die Methansulfonyloxy-, Trifluormethansulfonyloxy-, Methansulfonylamino-, Trifluormethansulfonylamino-, Methansulfonyl-methylamino-, Trifluormethansulfonyl-methylamino-, Methylsulfenylmethyl-, Methylsulfinylmethyl-, Methylsulfonylmethyl-, Cyan-, Aminocarbonyl-, Aminosulfonyl-, Methylaminosulfonyl- oder Dimethylaminosulfonylgruppe und

$R_2$ die Methoxy- oder Dimethylaminogruppe bedeuten, insbesondere jedoch diejenigen Verbindungen der obigen allgemeinen Formel Ia, in der A und B zusammen mit den beiden dazwischenliegenden Kohlenstoffatomen eine Gruppe der Formel

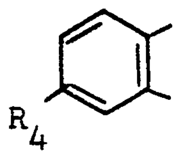 , 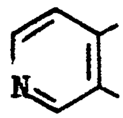 oder 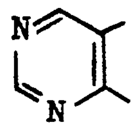 ,wobei

$R_4$ ein Wasserstoffatom, eine Hydroxy-, eine Cyan-, Aminocarbonyl- oder Acetylaminogruppe darstellt, $R_1$ die Methansulfonyloxy-, Methansulfonylamino-, N-Methyl-methansulfonylamino- oder Trifluormethansulfonyloxygruppe und $R_2$ die Methoxygruppe bedeuten, deren Tautomeren und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

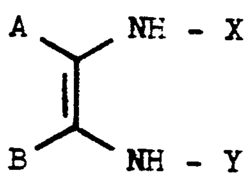 ,(II)

in der A und B wie eingangs definiert sind, einer der Reste X oder Y ein Wasserstoffatom und der andere der beiden Reste X und Y oder beide Reste X und Y eine Gruppe der Formel

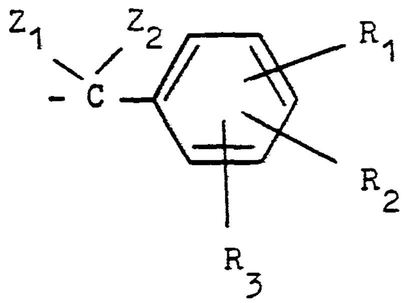

darstellen, in der

$R_1$ bis $R_3$ wie eingangs definiert sind,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester, Amid oder Methojodid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder Kondensationsmittel durchgeführt werden.

b) Zur Herstellung von Verbindungen der allgemeinen Formel I, in der $R_1$ eine Alkylsulfinylmethyl- oder Alkylsulfonylmethylgruppe darstellt:

Oxidation einer Verbindung der allgemeinen Formel

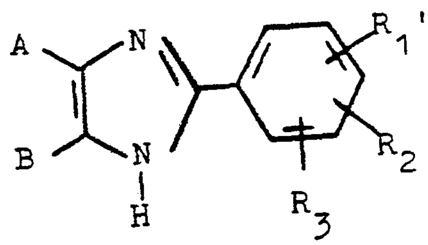

,(III)

in der
A, B, $R_2$ und $R_3$ wie eingangs definiert sind und
$R_1$' eine Alkylsulfenylmethyl- oder Alkylsulfinylmethyl- gruppe mit jeweils 1 bis 3 Kohlenstoffatomen im Alkyl- teil darstellt.

Die Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer Alkylsulfinylmethylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Äthanol bei -15 bis 25°C, mit Brom in Eisessig oder wässriger Essigsäure, mit N-Brom-succinimid in Äthanol, mit tert.Butyl-hyprochlorit in Methanol bei -80 bis

-30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol hydrolysiert.

Zur Herstellung einer Alkylsulfonylmethylverbindung der allgemeinen Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

c) Zur Herstellung von Verbindungen der allgemeinen Formel I, in der $R_1$ eine Alkansulfonyloxy-, Trifluormethansulfonyloxy-, Alkansulfonylamino-, N-Alkyl-alkansulfonylamino-, Trifluormethansulfonylamino- oder N-Alkyltrifluormethansulfonylaminogruppe und/oder $R_4$ eine Alkansulfonylamino- oder N-Alkyl-alkansulfonylaminogruppe darstellen:
Umsetzung einer Verbindung der allgemeinen Formel

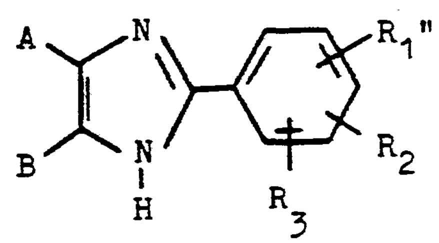

,(IV)

in der
$R_2$, $R_3$, A und B wie eingangs definiert sind und
$R_1$" eine Hydroxy-, Amino- oder N-Alkylaminogruppe mit 1 bis 3 Kohlenstoffatomen im Alkylteil darstellt, mit einer Sulfonsäure der allgemeinen Formel

$R_7 - SO_2OH$ ,(V)

in der $R_7$ eine Alkylgruppe mit 1 bis 3 Kohlenstoffatomen oder eine Trifluormethylgruppe darstellt, in Gegenwart eines wasserentziehenden und/oder die Säure oder das Amin aktivierenden Mittels oder mit deren reaktionsfähigen Derivaten.

Die Umsetzung wird zweckmäßigerweise in einem Lösungsmittel oder Lösungsmittelgemisch wie Methylenchlorid, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel V, z.B. mit deren Anhydrid oder Halogenid wie Methansulfonsäurechlorid oder Ethansulfonsäurechlorid, vorzugsweise bei Temperaturen zwischen 0 und 100°C, z.B. bei Temperaturen zwischen Raumtemperatur und 50°C, durchgeführt.

d) Zur Herstellung von Verbindungen der allgemeinen Formel I, in der $R_1$ eine durch eine Amino-, Alkylamino- oder Dialkylaminogruppe substituierte Carbonyl- oder Sulfonylgruppe und/oder $R_4$ eine Aminocarbonyl-, Alkylaminocarbonyl- oder Dialkylaminocarbonylgruppe darstellen:

Umsetzung einer Verbindung der allgemeinen Formel

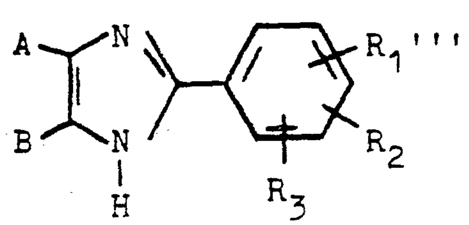

,(VI)

in der
$R_2$, $R_3$, A und B wie eingangs definiert sind und
$R_1'''$ eine Carboxyl- oder Hydroxysulfonylgruppe darstellt, oder eines reaktionsfähigen Derivates hiervon mit einem Amin der allgemeinen Formel

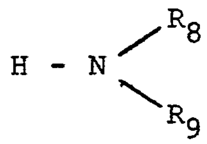

(VII)

in der
$R_8$ und $R_9$, die gleich oder verschieden sein können, Wasserstoffatome oder Alkylgruppen mit 1 bis 5 Kohlenstoffatome darstellen, oder mit einem reaktionsfähigen Derivat hiervon, falls $R_1'''$ die Carboxyl- oder Hydroxysulfonylgruppe und/oder $R_4$ die Carboxygruppe darstellt.

Die Umsetzung wird zweckmäßigerweise in einem Lösungsmitel oder Lösungsmittelgemisch wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phorphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung in einem entsprechenden Halogenid, z.B. dem Carbonsäure- oder Sulfonsäurechlorid, und einem entsprechenden Amin, wobei dieses gleichzeitig als Lösungsmittel dienen kann, und bei Temperaturen zwischen 0 und 50°C durchgeführt.

Bedeutet $R_4$ in einer Verbindung der allgemeinen Formel VI die Carboxygruppe, so wird diese gleichzeitig in eine entsprechende Aminocarbonyl-, Alkylaminocarbonyl- oder Dialkylaminocarbonylgruppe übergeführt.

Erhält man eine Verbindung der allgemeinen Formel I, in der $R_1$ und/oder $R_4$ die Cyangruppe darstellt, so kann diese anschließend mittels Alkoholyse und/oder Hydrolyse in eine entsprechende Verbindung, in der $R_1$ eine Alkoxycarbonylgruppe mit insgesamt 2 bis 5 Kohlenstoffatomen, die Aminocarbonyl- oder Carboxylgruppe und/oder $R_4$ eine Alkoxycarbonylgruppe mit insgesamt 2 bis 4 Kohlenstoffatomen, die Aminocarbonyl- oder Carboxylgruppe darstellen, übergeführt werden und/oder

eine Verbindung der allgemeinen Formel I, in der $R_1$ und/oder $R_4$ die Carboxygruppe darstellt, mittels Veresterung in eine entsprechende Verbindung der allgemeinen Formel I, in der $R_1$ eine Alkoxycarbonylgruppe mit insgesamt 2 bis 5 Kohlenstoffatomen und/oder $R_4$ eine Alkoxycarbonylgruppe mit insgesamt 2 bis 4 Kohlenstoffatomen darstellen, übergeführt werden und/oder

eine Verbindung der allgemeinen Formel I, in der $R_4$ eine Alkanoylaminogruppe darstellt, mittels Hydrolyse in eine entsprechende Verbindung der allgemeinen Formel I, in der $R_4$ eine Aminogruppe darstellt, übergeführt werden und/oder

eine Verbindung der allgemeinen Formel I, in der $R_4$ eine Nitrogruppe darstellt, mittels Reduktion in eine entsprechende Verbindung der allgemeinen Formel I, in der $R_4$ eine Aminogruppe darstellt, übergeführt werden und/oder

eine Verbindung der allgemeinen Formel I, in der $R_4$ eine Aminogruppe darstellt, mittels Überführung in ein Diazoniumsalz und anschließendem Erhitzen in eine entsprechende Verbindung der allgemeinen Formel I, in der $R_4$ die Hydroxygruppe darstellt, übergeführt werden und/oder

eine Verbindung der allgemeinen Formel I, in der $R_4$ eine Aminogruppe darstellt, mittels Carbamoylierung in eine entsprechende Verbindung der allgemeinen Formel I, in der $R_4$ eine Aminocarbonylamino- oder Alkylaminocarbonylaminogruppe darstellt, übergeführt werden und/oder

eine Verbindung der allgemeinen Formel I, in der $R_4$ eine Alkoxycarbonylgruppe darstellt, mittels Amidierung in eine entsprechende Verbindung der allgemeinen Formel I, in der $R_4$ eine Aminocarbonyl-, Alkylaminocarbonyl- oder Dialkylaminocarbonylgruppe darstellt, übergeführt werden und/oder

eine Verbindung der allgemeinen Formel I, in der $R_4$eine Alkoxycarbonylgruppe darstellt, mittels Reduktion in eine entsprechende Verbindung der allgemeinen Formel I, in der $R_4$ eine Hydroxymethylgruppe darstellt, übergeführt werden.

Die nachträgliche Alkoholyse und/oder Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittels wie Wasser, Wasser/Methanol, Ethanol, Wasser/Ethanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Die nachträgliche Veresterung wird zweckmäßigerweise in einem geeigneten Lösungsmittel, z.B. in einem entsprechenden

Alkohol, Pyridin, Toluol, Methylenchlorid, Tetrahydrofuran oder Dioxan, in Gegenwart eines säureaktivierenden und/oder wasserentziehenden Mittels wie Thionylchlorid, Chlorameisensäureäthylester, N,N'-Carbonyldiimidazol oder N,N'-Dicyclohexylcarbodiimid oder dessen Isoharnstoffethern, gegebenenfalls in Gegenwart eines Reaktionsbeschleunigers wie Kupfer-chlorid, oder durch Umesterung, z.B. mit einem entsprechenden Kohlensäurediester, bei Temperaturen zwischen 0 und 100°C, vorzugsweise jedoch bei Temperaturen zwischen 20°C und der Siedetemperatur des betreffenden Lösungsmittels, durchgeführt.

Die nachträgliche Reduktion der Nitrogruppe wird vorzugsweise in einem Lösungsmittel wie Wasser, Wasser/Ethanol, Methanol, Eisessig, Essigsäureethylester oder Dimethylformamid zweckmäßigerweise mit Wasserstoff in Gegenwart eines Hydrierungskatalysators wie Raney-Nickel, Platin oder Palladium/-Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfid oder Natriumdithionit, oder mit Hydrazin in Gegenwart von Raney-Nickel bei Temperaturen zwischen 0 und 50°C, vorzugsweise jedoch bei Raumtemperatur, durchgeführt.

Das nachträgliche Erhitzen eines Diazoniumsalzes, vorzugsweise des Hydrochlorids oder des Hydrosulfats, erfolgt vorzugsweise in einem wässrigen Lösungsmittel wie Wasser/Ethanol, Wasser/Tetrahydrofuran oder Wasser/Dioxan bei erhöhten Temperaturen, z.B. bei der Siedetemperatur des verwendeten Lösungsmittels. Das hierzu erforderliche Diazoniumsalz wird zweckmäßigerweise in einem geeigneten Lösungsmittel, z.B. in Wasser/Salzsäure, Methanol/Salzsäure, Ethanol/Salzsäure oder Dioxan/Salzsäure, durch Diazotierung einer entsprechenden Verbindung mit einem Nitrit, z.B. Natriumnitrit oder einem Ester der salpetrigen Säure, bei niederen Temperaturen, z.B. bei Temperaturen zwischen -10 und 5°C, hergestellt.

Die nachträgliche Carbamoylierung wird in einem inerten Lösungsmittel wie Wasser, Methylenchlorid, Tetrahydrofuran oder Dioxan mit einem entsprechenden Isocyanat wie Methylisocyanat oder Kaliumisocyanat in Gegenwart einer Säure wie Essigsäure bei Temperaturen zwischen 0 und 50°C durchgeführt.

Die nachträgliche Amidierung wird mit einem entsprechenden Amin gegebenenfalls in einem Druckgefäß zweckmäßigerweise in einem Lösungsmittel wie Wasser, Methylenchlorid, Chloroform, Tetrachlorkohlenstoff, Ether, Tetrahydrofuran, Dioxan, Benzol, Toluol oder Dimethylformamid bei Temperaturen zwischen 50 und 150°C durchgeführt.

Die nachträgliche Reduktion der Alkoxycarbonylgruppe wird vorzugsweise mit einem Metallhydrid, z.B. mit einem komplexen Metallhydrid wie Lithiumaluminiumhydrid, in einem geeigneten Lösungsmittel wie Diethyläther, Tetrahydrofuran oder Dioxan bei Temperaturen zwischen 0 und 100°C, vorzugsweise jedoch bei Temperaturen zwischen 20 und 60°C, durchgeführt.

Ferner können die erhaltenen Verbindungen der allgemeinen Formel I anschließend gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Furmarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis VII sind teilweise literaturbekannt bzw. erhält man nach literaturbekannten Verfahren.

So erhält man beispielsweise die als Ausgangsstoffe verwendeten Verbindungen der allgemeinen Formel II durch Acylierung der entsprechenden 4,5-Diaminoverbindungen und die Verbindungen der allgemeinen Formel III, IV und VI durch anschließende Kondensation mit einem entsprechenden Benzoesäurederivat und gegebenenfalls anschließende Oxidation und/oder Acylierung (siehe BE-PS 810.545 und EP-A-0.024.290).

Wie bereits eingangs erwähnt weisen die neuen Verbindungen der allgemeinen Formel I, deren 1H Tautomere und deren physiologisch verträgliche Säureadditionssalze bei einer langen Wirkungsdauer überlegene pharmakologische Eigenschaften auf, insbesondere eine blutdrucksenkende und/oder positiv-inotrope Wirkung.

Beispielweise wurden die Verbindungen

A = 2-(2'-Methoxy-4'-cyan-phenyl)-imidazo[4,5-b]pyridin,

B = 2-(2'-Methoxy-4'-aminocarbonyl-phenyl)-imidazo[4,5-b]-pyridin,

C = 8-(2'-Methoxy-4'-cyan-phenyl)-purin,

D = 8-(2'-Methoxy-4'-aminocarbonyl-phenyl)-purin,

E = 2-(2'-Methoxy-4'-methylaminosulfonyl-phenyl)-imidazo-[4,5-b]pyridin,

F = 8-(2'-Methoxy-4'-methylaminosulfonyl-phenyl)-purin,

G = 2-(2'-Methoxy-4'-methansulfonyloxy-phenyl)-imidazo-[4,5-b]pyridin,

H = 2-(2'-Methoxy-4'-methansulfonyloxy-phenyl)-benzimidazol,

I = 8-(2'-Methoxy-4'-methansulfonyloxy-phenyl)-purin,

K = 8-(2'-Methoxy-4'-methansulfonylamino-phenyl)-purin,

L = 2-(2'-Methoxy-4'-N-methyl-methansulfonylamino-phenyl)-imidazo[4,5-b]pyridin,

M = 8-(2'-Methoxy-4'-N-methyl-methansulfonylamino-phenyl)-purin,

N = 2-(2'-Methoxy-4'-methansulfonylamino-phenyl)-benzimidazol,

O = 2-(2'-Methoxy-4'-methylthiomethyl-phenyl)-imidazo-[4,5-b]pyridin,

P = 5-Cyano-2-(4'-methansulfonyloxy-2'-methoxy-phenyl)-benzimidazol,

Q = 5-Acetylamino-2-(4'-methansulfonyloxy-2'-methoxy-phenyl)-benzimidazol,

R = 5-Amino-2-(4'-methansulfonylamino-2'-methoxy-phenyl)-benzimidazol,

S = 8-(2'-Methoxy-4'-trifluormethansulfonyloxy-phenyl)-purin,

T = 2-(4'-Methansulfonylamino-2'-methoxy-phenyl)-imidazo-[4,5-c]pyridin,

U = 2-(4'-Methansulfonyloxy-2'-methoxy-phenyl)-imidazo-[4,5-c]pyridin und

V = 5-Hydroxy-2-(4'-methansulfonylamino-2'-methoxy-phenyl)-benzimidazol

auf ihre biologischen Eigenschaften wie folgt untersucht:

1.) Bestimmung der Blutdruckwirkung und der positiv inotropen Wirkung an der narkotisierten Katze

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparamter $dp/dt_{max}$ mittels eines Analogdifferenzierers gewonnen. Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 2 mg/kg i.v..

Die nachfolgende Tabelle enthält die Mittelwerte:

| Substanz | Dosis mg/kg i.v. | Zunahme von dp/dt$_{max}$ in % | Blutdrucksenkung in mm Hg |
|---|---|---|---|
| A | 2,0 | + 76 | - 41/-50 |
| B | 2,0 | + 99 | - 9/-19 |
| C | 2,0 | + 106 | - 25/-34 |
| D | 2,0 | + 103 | - 20/-32 |
| E | 2,0 | + 72 | - 37/-33 |
| F | 2,0 | + 124 | - 57/-32 |
| G | 2,0 | + 148 | - 42/-40 |
| H | 2,0 | + 94 | - 24/-36 |
| I | 2,0 | + 89 | - 18/-50 |
| K | 2,0 | + 79 | - 27/-35 |
| L | 2,0 | + 77 | - 30/-42 |
| M | 2,0 | + 69 | - 37/-47 |
| N | 2,0 | + 72 | - 18/-30 |
| O | 2,0 | + 73 | - 24/-26 |
| P | 2,0 | + 120 | - 55/-55 |
| Q | 2,0 | + 78 | - 12/-32 |
| R | 2,0 | + 107 | - 30/-37 |
| S | 2,0 | + 48 | - 16/-32 |
| T | 2,0 | + 112 | - 22/-44 |
| U | 2,0 | + 167 | - 27/-27 |
| V | 2,0 | + 85 | - 8/-28 |

2. <u>Akute Toxizität:</u>

Die akute Toxizität der zu untersuchenden Substanzen wurde orientierend an Gruppen von je 10 Mäusen nach oraler Gabe einer Einzeldosis von 300 mg/kg bestimmt (Beobachtungszeit: 14 Tage):

| Substanz | | Orientierend akute Toxizität |
| --- | --- | --- |
| G | > | 300 mg/kg (0 von 10 Tieren gestorben) |
| N | > | 300 mg/kg (0 von 10 Tieren gestorben) |

Die neuen Verbindungen sind gut verträglich, so konnte bei der Untersuchung der Substanzen A bis V keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern und durch die Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,3 - 2,2 mg/kg Körpergewicht, vorzugsweise jedoch 0,7 - 1,5 mg/kg Körpergewicht.

Die nachfolgenden Beispiele sollen die Erfindung näher erläutern:

Beispiel A

2-(2'-Methoxy-4'-benzyloxy-phenyl)-benzimidazol

5,2 g (20 mMol) 2-Methoxy-4-benzyloxy-benzoesäure und 2,2 g (20 mMol) o-Phenylendiamin werden miteinander verrieben, mit 50 ml Phosphoroxychlorid versetzt und das Gemisch 1,5 Stunden lang zum Rückfluß erhitzt. Nach dem Abkühlen wird die Lösung auf 300 g Eis gegeben und mit 30%iger Natronlauge alkalisch gestellt. Das ausgefallene Produkt wird abgesaugt, mit Wasser gewaschen und im Umlufttrockenschrank bei 50°C getrocknet.
Ausbeute: 3,5 g (53 % der Theorie),
$R_F$-Wert: 0,8 (Kieselgel, Laufmittel: Methylenchlorid/Ethanol = 19/1).

Beispiel B

2-(2'-Methoxy-4'-hydroxy-phenyl)-benzimidazol

3,3 g (10 mMol) 2-(2'-Methoxy-4'-benzyloxy-phenyl)-benzimidazol werden in 400 ml Ethanol gelöst und nach Zugabe von 0,5 g Palladium auf Kohle (20 %) in einer Parr-Apparatur bei 50°C mit Wasserstoff (5 bar) behandelt. Nach vollständiger Wasserstoffaufnahme wird vom Katalysator abgesaugt und das Filtrat eingeengt. Der feste Rückstand wird mit Ether verrieben, abgesaugt und getrocknet.
Ausbeute: 2,3 g (95,8 % der Theorie),
$R_F$-Wert: 0,36 (Kieselgel, Laufmittel: Methylenchlorid/Ethanol = 19/1).

Beispiel C

2-(2'-Methoxy-4'-chlorsulfonyl-phenyl)-imidazo[4,5-b]pyridin-hydrochlorid

5 g 2-Methoxy-4-chlorsulfonyl-benzoesäure (hergestellt aus 4-Amino-2-methoxy-benzoesäure über die entsprechende Diazoniumverbidung) werden in 400 ml Phosphoroxychlorid gelöst und 30 Minuten auf 80°C erwärmt. Danach werden 3,6 g 2,3-Diamino-pyridin zugegeben und dann 4 Stunden zum Rückfluß erhitzt. Das Reaktionsgemisch wird im Vakuum eingedampft, mit Toluol nachgedampft und der feste Rückstand ohne weitere Reinigung weiter verarbeitet.

Beispiel D

2-(2'-Methoxy-4'-chlorsulfonyl-phenyl)-purin-hydrochlorid

Hergestellt analog Beispiel C aus 2,5 g 2-Methoxy-4-chlorsulfonyl-benzoesäure und 2,4 g 4,5-Diamino-pyrimidin (Kristallisat aus Dihydrochlorid und 1 Mol Kochsalz) durch achtstündiges Kochen unter Rückfluß. Das feste Rohprodukt wird ohne weitere Reinigung weiter verarbeitet.

Beispiel E

2-(2'-Dimethylamino-4'-amino-phenyl)-imidazo[4,5-b]pyridin-dihydrochlorid

2,83 g 2-(2'-Dimethylamino-4'-nitro-phenyl)-imidazo[4,5-b]-pyridin werden in einer Mischung aus 100 ml Methanol, 10 ml konzentrierter methanolischer Salzsäure und 1 g Palladium/-Kohle 4,5 Stunden mit Wasserstoffgas von 5 bar bei Raumtemperatur hydriert. Der Katalysator wird dann abfiltriert,

das Filtrat eingedampft, der Rückstand mit Aceton kristallin gerieben, abfiltriert und mit einer Ether-Aceton-Mischung gewaschen.
Ausbeute: 2,3 g (70 % der Theorie),
Schmelzpunkt: 208-210°C.

Beispiel F

2-(2'-Dimethylamino-4'-hydroxy-phenyl)-imidazo[4,5-b]pyridin-dihydrochlorid

1,15 g 2-(2'-Dimethylamino-4'-amino-phenyl)-imidazo[4,5-b]-pyridin werden in 25 ml Eiswasser suspendiert, mit 2,5 ml konzentrierter Schwefelsäure versetzt und auf -5°C abgekühlt. Danach wird eine Lösung von 0,35 g Natriumnitrit in 5 ml Wasser langsam zugetropft und 15 Minuten bei 0°C nachgerührt. Die entstandene organgegelbe Suspension wird langsam in eine 90°C warme Lösung aus 6 ml konzentrierter Schwefelsäure und 12 ml Wasser eingetropft. Danach wird auf ein Viertel des Volumens eingedampft und mit wäßrigem Ammoniak neutralisiert. Der ausgefallene Niederschlag wird abgesaugt und nach Trocknen in Methanol aufgenommen, woraus mit etherischer Salzsäure das Dihydrochlorid gefällt wird.
Ausbeute: 0,63 g (42 % der Theorie).
Schmelzpunkt: 212-214°C.

Beispiel 1

2-(2'-Methoxy-4'-methansulfonyloxy-phenyl)-benzimidazol

2,2 g (9 mMol) 2-(2'-Methoxy-4'-hydroxy-phenyl)-benzimidazol werden in 40 ml Pyridin suspendiert und unter Rühren 2 ml Methansulfonsäurechlorid bei Raumtemperatur zugetropft. Anschließend wird 1 Stunde lang auf 50°C erwärmt, dann mit 10 ml Wasser versetzt und die Lösung im Vakuum bis zur Trockne eingeengt. Der feste Rückstand wird mit Wasser verrieben, abgesaugt und aus Ethanol umkristallisiert.
Ausbeute: 1,2 g (41,9 % der Theorie),
$R_F$-Wert: 0,67 (Kieselgel, Laufmittel: Methylenchlorid/Ethanol = 19/1).
Schmelzpunkt: 197-198°C.
$C_{15}H_{14}N_2O_4S$ (318,34)

| | | | | | | | | |
|---|---|---|---|---|---|---|---|---|
| Ber.: | C | 56,59 | H | 4,43 | N | 8,80 | S | 10,07 |
| Gef.: | | 56,40 | | 4,43 | | 8,74 | | 10,20 |

Beispiel 2

2-(2'-Methoxy-4'-methansulfonyloxy-phenyl)-imidazo[4,5-b]-pyridin

Hergestellt analog Beispiel 1 aus 2-(2'-Methoxy-4'-hydroxy-phenyl)-imidazo[4,5-b]pyridin und Methansulfonsäurechlorid.
Ausbeute: 67,3 % der Theorie,
Schmelzpunkt: 208-209°C.
$C_{14}H_{13}N_3O_4S$ (319,3)

| | | | | | | | | |
|---|---|---|---|---|---|---|---|---|
| Ber.: | C | 52,66 | H | 4,10 | N | 13,16 | S | 10,04 |
| Gef.: | | 52,41 | | 3,98 | | 13,22 | | 9,99 |

Beispiel 3

2-(2',4'-Dimethoxy-3'-methansulfonyloxy-phenyl)-imidazo-[4,5-b]pyridin-hydrochlorid

Hergestellt analog Beispiel 1 aus 2-(2',4'-Dimethoxy-3'-hydroxy-phenyl)-imidazo[4,5-b]pyridin und Methansulfonsäurechlorid.
Ausbeute: 58,4 % der Theorie,
Schmelzpunkt: 202-206°C (Zersetzung).
$C_{15}H_{16}ClN_3O_5S$ (385,83)

| | | | | | | | | |
|---|---|---|---|---|---|---|---|---|
| Ber.: | C | 46,69 | H | 4,18 | N | 10,89 | Cl | 9,19 |
| Gef.: | | 46,83 | | 4,13 | | 11,18 | | 9,47 |

Beispiel 4

8-(2'-Methoxy-4'-methansulfonyloxy-phenyl)-purin

Hergestellt analog Beispiel 1 aus 8-(2'-Methoxy-4'-hydroxy-phenyl)-purin und Methansulfonsäurechlorid.
Ausbeute: 46,9 % der Theorie,
Schmelzpunkt: 225-227°C.
$C_{13}H_{12}N_4O_4S$ (320,3)

| | | | | | | | | |
|---|---|---|---|---|---|---|---|---|
| Ber.: | C | 48,75 | H | 3,78 | N | 17,49 | S | 10,01 |
| Gef.: | | 48,52 | | 3,72 | | 17,25 | | 10,00 |

Beispiel 5

8-(2'-Methoxy-4'-ethansulfonyloxy-phenyl)-purin

Hergestellt analog Beispiel 1 aus 8-(2'-Methoxy-4'-hydroxy-

 

phenyl)-purin und Ethansulfonsäurechlorid.
Ausbeute: 12 % der Theorie,
Schmelzpunkt: 195-196°C.

$C_{14}H_{14}N_4O_4S$ (334,4)

| | | | | | | | | |
|---|---|---|---|---|---|---|---|---|
| Ber.: | C | 50,29 | H | 4,22 | N | 16,76 | S | 9,59 |
| Gef.: | | 50,02 | | 4,15 | | 16,59 | | 9,83 |

Beispiel 6

2-(2'-Methoxy-4'-ethansulfonyloxy-phenyl)-imidazo[4,5-b]-pyridin

Hergestellt analog Beispiel 1 aus 2-(2'-Methoxy-4'-hydroxy-phenyl)-imidazo[4,5-b]pyridin und Ethansulfonsäurechlorid.
Ausbeute: 20,1 % der Theorie,
Schmelzpunkt: 206-209°C.

$C_{15}H_{15}N_3O_4S$ (333,4)

| | | | | | | | | |
|---|---|---|---|---|---|---|---|---|
| Ber.: | C | 54,04 | H | 4,54 | N | 12,60 | S | 9,62 |
| Gef.: | | 54,11 | | 4,59 | | 12,43 | | 9,71 |

Beispiel 7

2-(2'-Methoxy-3'-methansulfonyloxy-phenyl)-imidazo[4,5-b]-pyridin

Hergestellt analog Beispiel 1 aus 2-(2'-Methoxy-3'-hydroxy-phenyl)-imidazo[4,5-b]pyridin und Methansulfonsäurechlorid.
Ausbeutre: 70,7 % der Theorie,
Schmelzpunkt: 153-155°C.

$C_{14}H_{13}N_3O_4S$ (319,3)

| | | | | | | | | |
|---|---|---|---|---|---|---|---|---|
| Ber.: | C | 52,66 | H | 4,10 | N | 13,16 | S | 10,04 |
| Gef.: | | 52,40 | | 3,96 | | 13,17 | | 10,04 |

## Beispiel 8

8-(2'-Methoxy-3'-methansulfonyloxy-phenyl)-purin

Hergestellt analog Beispiel 1 aus 8-(2'-Methoxy-3'-hydroxy-phenyl)-purin und Methansulfonsäurechlorid.
Ausbeute: 46,8 % der Theorie,
Schmelzpunkt: 187-188°C.
$C_{13}H_{12}N_4O_4S$ (320,3)

| | | | | | | | | |
|---|---|---|---|---|---|---|---|---|
| Ber.: | C | 48,75 | H | 3,78 | N | 17,50 | S | 10,01 |
| Gef.: | | 48,70 | | 4,02 | | 17,37 | | 10,35 |

## Beispiel 9

2-(3'-Methoxy-5'-methansulfonyloxy-phenyl)-imidazo[4,5-b]-pyridin

Hergestellt analog Beispiel 1 aus 2-(3'-Methoxy-5'-hydroxy-phenyl)-imidazo[4,5-b]pyridin und Methansulfonsäurechlorid.
Ausbeute: 15 % der Theorie,
Schmelzpunkt: 225-227°C.
$C_{14}H_{13}N_3O_4S$ (319,35)

| | | | | | | | | |
|---|---|---|---|---|---|---|---|---|
| Ber.: | C | 52,65 | H | 4,10 | N | 13,15 | S | 10,04 |
| Gef.: | | 52,86 | | 4,32 | | 13,20 | | 9,91 |

## Beispiel 10

2-(3'-Methoxy-4'-methansulfonyloxy-phenyl)-imidazo[4,5-b]-pyridin

Hergestellt analog Beispiel 1 aus 2-(3'-Methoxy-4'-hydroxy-phenyl)-imidazo[4,5-b]pyridin und Methansulfonsäurechlorid.

Ausbeute: 43,8 % der Theorie,
Schmelzpunkt: 235-237°C.

$C_{14}H_{13}N_3O_4S$ (319,3)

| | | | | | | | | |
|---|---|---|---|---|---|---|---|---|
| Ber.: | C | 52,66 | H | 4,10 | N | 13,16 | S | 10,04 |
| Gef.: | | 52,80 | | 4,05 | | 13,11 | | 10,15 |

Beispiel 11

5-Trifluormethyl-2-(2'-methoxy-4'-methansulfonyloxy-phenyl)-benzimidazol

Hergestellt analog Beispiel 1 aus 5-Trifluormethyl-2-(2'-methoxy-4'-hydroxy-phenyl)-benzimidazol und Methansulfonsäurechlorid.

Ausbeute: 50,8 % der Theorie,
Schmelzpunkt: 138-140°C.

$C_{16}H_{13}F_3N_2O_4S$ (386,36)

| | | | | | | | | |
|---|---|---|---|---|---|---|---|---|
| Ber.: | C | 49,74 | H | 3,39 | N | 7,25 | S | 8,30 |
| Gef.: | | 49,43 | | 3,54 | | 7,17 | | 8,34 |

Beispiel 12

5-Methoxy-2-(2'-methoxy-4'-methansulfonyloxy-phenyl)-benzimidazol

Hergestellt analog Beispiel 1 aus 5-Methoxy-2-(2'-methoxy-4'-hydroxy-phenyl)-benzimidazol und Methansulfonsäurechlorid.

Ausbeute: 88,2 % der Theorie,
Schmelzpunkt: 152-154°C.

$C_{16}H_{16}N_2O_5S$ (348,39)

| | | | | | | | | |
|---|---|---|---|---|---|---|---|---|
| Ber.: | C | 55,16 | H | 4,63 | N | 8,04 | S | 9,20 |
| Gef.: | | 55,38 | | 4,78 | | 7,94 | | 9,28 |

Beispiel 13

5-Cyano-2-(2'-methoxy-4'-methansulfonyloxy-phenyl)-benzimidazol

Hergestellt analog Beispiel 1 aus 5-Cyano-2-(2'-methoxy-4'-hydroxy-phenyl)-benzimidazol und Methansulfonsäurechlorid.
Ausbeute: 23,8 % der Theorie,
Schmelzpunkt: 225-227°C.
$C_{16}H_{13}N_3O_4S$ (343,37)

| | | | | | | | | |
|---|---|---|---|---|---|---|---|---|
| Ber.: | C | 55,96 | H | 3,82 | N | 12,24 | S | 9,34 |
| Gef.: | | 55,71 | | 3,93 | | 12,08 | | 9,24 |

Beispiel 14

2-(2'-Methoxy-4'-methansulfonylamino-phenyl)-imidazo-[4,5-c]pyridin

5,45 g (50 mMol) 3,4-Diaminopyridin und 12,25 g (50 mMol) 2-Methoxy-4-methansulfonylamino-benzoesäure werden miteinander verrieben und in 300 ml Phosphoroxychlorid vier Stunden lang zum Rückfluß erhitzt. Das überschüssige Phosphoroxychlorid wird dann abdestilliert, der Rückstand mit 500 ml Wasser versetzt und mit konzentriertem Ammoniak auf pH 8 eingestellt. Von unlöslichen Bestandteilen wird abfiltriert, das Filtrat mit Natriumchlorid gesättigt, wobei das Rohprodukt ausfällt. Nach der chromatographischen Reinigung (800 g Aluminiumoxid, Elutionsmittel: Dichlormethan mit 5 bis 10 % Ethanol) erhält man 4,8 g (25,2 % der Theorie).
Schmelzpunkt: > 250°C,
$C_{14}H_{14}N_4O_3S$ (318,4)

| | | | | | | |
|---|---|---|---|---|---|---|
| Ber.: | C | 52,81 | H | 4,43 | N | 17,60 |
| Gef.: | | 52,61 | | 4,63 | | 17,35 |

$^1$H-NMR-Spektrum (DMSO-$d_6$/$CD_3OD$):

δ = 3,2 (s,2H); 4,1 (s,3H); 6,9-7,3 (m,2H); 7,5-7,8 (m,1H); 8,2-8,5 (m,2H); 8,9-9,0 (breites s, 1H) ppm.

Analog wurden folgende Verbindungen hergestellt:

2-(2'-Methoxy-4'-N-methyl-methansulfonylamino-phenyl)-imidazo[4,5-c]pyridin

Hergestellt aus 3,4-Diaminopyridin und 2-Methoxy-4-N-methyl-methansulfonylamino-benzoesäure.

Ausbeute: 19,8 % der Theorie,

Schmelzpunkt: > 250°C.

$C_{15}H_{16}N_4O_3S$ (332,4)

| | | | | | | |
|---|---|---|---|---|---|---|
| Ber.: | C | 54,20 | H | 4,85 | N | 16,86 |
| Gef.: | | 54,47 | | 4,91 | | 16,62 |

$^1$H-NMR-Spektrum (DMSO-$d_6$/$CD_3OD$):

δ = 3,1 (s,3H); 3,4 (s,3H); 4,1 (s,3H); 6,9-7,3 (m,2H); 7,5-7,8 (m,1H); 8,2-8,5 (m,2H); 8,9-9,0 (breites s, 1H) ppm.

2-(2'-Methoxy-4'-N-ethyl-methansulfonylamino-phenyl)-imidazo-[4,5-c]pyridin

Hergestellt aus 3,4-Diaminopyridin und 2-Methoxy-4-N-ethyl-methansulfonyloxy-benzoesäure.

Ausbeute: 16,9 % der Theorie,

$C_{16}H_{18}N_4O_3S$ (346,40)

| | | | | | | |
|---|---|---|---|---|---|---|
| Ber.: | C | 55,47 | H | 5,24 | N | 16,18 |
| Gef.: | | 55,58 | | 5,31 | | 15,92 |

$^1$H-NMR-Spektrum (DMSO-$d_6$/$CD_3OD$):

δ = 1,0-1,3 (t,3H); 3,1 (s,3H); 3,6-4,0 (q,2H); 4,1 (s,3H); 6,9-7,3 (m,2H); 7,5-7,8 (m,1H); 8,2-8,5 (m,2H); 8,9-9,0 (breites s,1H) ppm.

Beispiel 15

2-(2'-Methoxy-4'-methansulfonylamino-phenyl)-imidazo[4,5-b]-pyridin

Hergestellt analog Beispiel 14 aus 2,3-Diaminopyridin und 2-Methoxy-4-methansulfonylamino-benzoesäure.
Ausbeute: 57,3 % der Theorie,
Schmelzpunkt: 236-238°C.
$R_F$-Wert: 0,50 (Kieselgel, Laufmittel: Methylenchlorid/Ethanol = 19/1).

Beispiel 16

8-(2'-Methoxy-4'-methansulfonylamino-phenyl)-purin

Hergestellt analog Beispiel 14 aus 4,5-Diaminopyrimidin und 2-Methoxy-4-methansulfonylamino-benzoesäure.
Ausbeute: 40,75 % der Theorie,
Schmelzpunkt: 237-238°C.

$C_{13}H_{13}N_5O_3S$

| | | | | | | | | |
|---|---|---|---|---|---|---|---|---|
| Ber.: | C | 48,89 | H | 4,10 | N | 21,93 | S | 10,04 |
| Gef.: | | 48,81 | | 4,37 | | 21,88 | | 9,95 |

Beispiel 17

2-(2'-Methoxy-4'-N-methyl-methansulfonylamino-phenyl)-imidazo[4,5-b]pyridin

Hergestellt analog Beispiel 14 aus 2,3-Diaminopyridin und 2-Methoxy-4-N-methyl-methansulfonylamino-benzoesäure.
Ausbeute: 57,2 % der Theorie,
Schmelzpunkt: 238-240°C.

$C_{15}H_{16}N_4O_3S$ (332,4)

| | | | | | | | | |
|---|---|---|---|---|---|---|---|---|
| Ber.: | C | 54,20 | H | 4,85 | N | 16,86 | S | 9,65 |
| Gef.: | | 54,20 | | 4,91 | | 16,68 | | 9,86 |

Beispiel 18

8-(2'-Methoxy-4'-N-methyl-methansulfonylamino-phenyl)-purin

Hergestellt analog Beispiel 14 aus 4,5-Diaminopyrimidin und 2-Methoxy-4-N-methyl-methansulfonylamino-benzoesäure.
Ausbeute: 45,5 % der Theorie,
Schmelzpunkt: > 250°C.
$C_{14}H_{15}N_5O_3S$ (333,4)

| | | | | | | | | |
|---|---|---|---|---|---|---|---|---|
| Ber.: | C | 50,44 | H | 4,54 | N | 21,01 | S | 9,62 |
| Gef.: | | 50,15 | | 4,77 | | 20,77 | | 9,50 |

Beispiel 19

2-(3'-Methoxy-4'-methansulfonylamino-phenyl)-imidazo[4,5-b]-pyridin

Hergestellt analog Beispiel 14 aus 2,3-Diaminopyridin und 3-Methoxy-4-methansulfonylamino-benzoesäure.
Ausbeute: 21,4 % der Theorie,
Schmelzpunkt: > 250°C.
$C_{14}H_{14}N_4O_3S$ (318,4)

| | | | | | | | | |
|---|---|---|---|---|---|---|---|---|
| Ber.: | C | 52,81 | H | 4,43 | N | 17,60 | S | 10,07 |
| Gef.: | | 52,60 | | 4,46 | | 17,94 | | 10,10 |

Beispiel 20

8-(3'-Methoxy-4'-methansulfonylamino-phenyl)-purin

Hergestellt analog Beispiel 14 aus 4,5-Diaminopyrimidin und

3-Methoxy-4-methansulfonylamino-benzoesäure.
Ausbeute: 11,2 % der Theorie,
Schmelzpunkt: > 250°C.
$C_{13}H_{13}N_5O_3S$ (319,35)

| | | | | | | | | |
|---|---|---|---|---|---|---|---|---|
| Ber.: | C | 48,89 | H | 4,10 | N | 21,93 | S | 10,04 |
| Gef.: | | 48,31 | | 4,45 | | 21,74 | | 10,80 |

Beispiel 21

2-(3'-Methoxy-4'-N-methyl-methansulfonylamino-phenyl)-imidazo[4,5-b]pyridin

Hergestellt analog Beispiel 14 aus 2,3-Diaminopyridin und 3-Methoxy-4-N-methyl-methansulfonylamino-benzoesäure.
Ausbeute: 38,8 % der Theorie,
Schmelzpunkt: > 250°C.
$C_{15}H_{16}N_4O_3S$ (332,39)

| | | | | | | | | |
|---|---|---|---|---|---|---|---|---|
| Ber.: | C | 54,20 | H | 4,85 | N | 16,85 | S | 9,65 |
| Gef.: | | 54,59 | | 5,22 | | 16,57 | | 9,55 |

Beispiel 22

8-(3'-Methoxy-4'-N-methyl-methansulfonylamino-phenyl)-purin

Hergestellt analog Beispiel 14 aus 4,5-Diaminopyridin und 3-Methoxy-4-N-methyl-methansulfonylamino-benzoesäure.
Ausbeute: 9,6 % der Theorie,
Schmelzpunkt: > 250°C.
$C_{14}H_{15}N_5O_3S$ (333,38)

| | | | | | | | | |
|---|---|---|---|---|---|---|---|---|
| Ber.: | C | 50,44 | H | 4,54 | N | 21,00 | S | 9,62 |
| Gef.: | | 50,71 | | 5,10 | | 20,58 | | 9,59 |

Beispiel 23

2-(2'-Methoxy-4'-methansulfonylamino-phenyl)-benzimidazol

Hergestellt analog Beispiel 14 aus o-Phenylendiamin und 2-Methoxy-4-methansulfonylamino-benzoesäure.
Ausbeute: 23,6 % der Theorie,
Schmelzpunkt: > 250°C.
$C_{15}H_{15}N_3O_3S$ (317,38)

| | | | | | | | | |
|---|---|---|---|---|---|---|---|---|
| Ber.: | C | 56,76 | H | 4,76 | N | 13,24 | S | 10,10 |
| Gef.: | | 56,40 | | 4,61 | | 12,96 | | 10,27 |

Beispiel 24

2-(2'-Methoxy-4'-N-methyl-methansulfonylamino-phenyl)-benzimidazol

Hergestellt analog Beispiel 14 aus o-Phenylendiamin und 2-Methoxy-4-N-methyl-methansulfonylamino-benzoesäure.
Ausbeute: 21,7 % der Theorie,
Schmelzpunkt: > 250°C.
$C_{16}H_{17}N_3O_3S$ (331,40)

| | | | | | | | | |
|---|---|---|---|---|---|---|---|---|
| Ber.: | C | 57,99 | H | 5,17 | N | 12,68 | S | 9,68 |
| Gef.: | | 57,98 | | 4,96 | | 12,79 | | 9,53 |

Beispiel 25

8-(2'-Methoxy-4'-N-ethyl-methansulfonylamino-phenyl)-purin

Hergestellt analog Beispiel 14 aus 4,5-Diaminopyrimidin und 2-Methoxy-4-N-ethyl-methansulfonylamino-benzoesäure.
Ausbeute: 13,8 % der Theorie,
Schmelzpunkt: 246-248°C.

$C_{15}H_{17}N_5O_3S$ (347,41)

| | | | | | | | | |
|---|---|---|---|---|---|---|---|---|
| Ber.: | C | 51,85 | H | 4,93 | H | 20,16 | S | 9,23 |
| Gef.: | | 51,83 | | 4,78 | | 19,90 | | 9,46 |

$^1$H-NMR-Spektrum (DMSO-$d_6$/$CD_3OD$):
δ = 1,0-1,3 (t,3H); 3,1 (s,3H); 3,6-4,0 (q,2H); 4,1 (s,3H); 7,1-7,3 (m,2H); 8,2-8,4 (d,1H); 8,8 (s,1H); 9,0 (s,1H) ppm.

Beispiel 26

5-Methoxy-2-(2'-methoxy-4'-methansulfonylamino-phenyl)-benzimidazol

Hergestellt analog Beispiel 14 aus 4-Methoxy-o-phenylendiamin und 2-Methoxy-4-methansulfonylamino-benzoesäure.
Ausbeute: 28,8 % der Theorie,
Schmelzpunkt: 195-198°C.
$C_{16}H_{17}N_3O_4S$ (347,40)

| | | | | | | | | |
|---|---|---|---|---|---|---|---|---|
| Ber.: | C | 55,32 | H | 4,93 | N | 12,10 | S | 9,23 |
| Gef.: | | 55,54 | | 5,34 | | 11,93 | | 8,70 |

Beispiel 27

5-Chlor-2-(2'-methoxy-4'-methansulfonylamino-phenyl)-benzimidazol

Hergestellt analog Beispiel 14 aus 4-Chlor-o-phenylendiamin und 2-Methoxy-4-methansulfonylamino-benzoesäure.

Ausbeute: 43,8 % der Theorie,
Schmelzpunkt: 230-232°C.
$C_{15}H_{14}ClN_3O_3S$ (351,82)

| | | | | | | | | | | |
|---|---|---|---|---|---|---|---|---|---|---|
| Ber.: | C | 51,21 | H | 4,01 | N | 11,94 | Cl | 10,08 | S | 9,11 |
| Gef.: | | 51,27 | | 4,02 | | 11,87 | | 10,15 | | 9,00 |

Beispiel 28

5-Chlor-2-(2'-methoxy-4'-N-methyl-methansulfonylamino-phenyl)-benzimidazol

Hergestellt analog Beispiel 14 aus 4-Chlor-o-phenylendiamin und 2-Methoxy-4-N-methyl-methansulfonylamino-benzoesäure.

Ausbeute: 28,8 % der Theorie,

Schmelzpunkt: 191-192°C.

$C_{16}H_{16}ClN_3O_3S$ (365,85)

| | | | | | | | | | | |
|---|---|---|---|---|---|---|---|---|---|---|
| Ber.: | C | 52,53 | H | 4,41 | N | 11,49 | Cl | 9,69 | S | 8,76 |
| Gef.: | | 52,95 | | 4,52 | | 11,45 | | 9,86 | | 8,82 |

Beispiel 29

2-(2'-Methoxy-4'-methylthiomethyl-phenyl)-imidazo[4,5-b]-pyridin

Hergestellt analog Beispiel 14 aus 2,3-Diaminopyridin und 2-Methoxy-4-methylthiomethyl-benzoesäure.

Ausbeute: 35,1 % der Theorie,

Schmelzpunkt: 148-149°C.

$C_{15}H_{15}N_3OS$ (285,35)

| | | | | | | | | |
|---|---|---|---|---|---|---|---|---|
| Ber.: | C | 63,14 | H | 5,30 | N | 14,73 | S | 11,24 |
| Gef.: | | 62,72 | | 5,53 | | 14,47 | | 10,84 |

Beispiel 30

8-(2'-Methoxy-4'-methylthiomethyl-phenyl)-purin

Hergestellt analog Beispiel 14 aus 4,5-Diaminopyrimidin und 2-Methoxy-4-methylthiomethyl-benzoesäure.

Ausbeute: 31,4 % der Theorie,

Schmelzpunkt: 194-196°C.

$C_{14}H_{14}N_4OS$ (286,36)

| | | | | | | |
|---|---|---|---|---|---|---|
| Ber.: | C | 58,72 | H | 4,93 | N | 19,57 |
| Gef.: | | 58,48 | | 4,87 | | 19,29 |

Beispiel 31

2-(2'-Methoxy-4'-methylsulfonylmethyl-phenyl)-imidazo-[4,5-b]pyridin

1,4 g (4,9 mMol) 2-(2'-Methoxy-4'-methylthiomethyl-phenyl)-imidazo[4,5-b]pyridin werden in 30 ml Eisesig gelöst und mit 3 ml 30%igem Wasserstoffperoxid versetzt. Nach 48 Stunden bei Raumtemperatur wird die Lösung mit 200 ml Wasser verdünnt, mit konzentrierter Ammoniak-Lösung alkalisch gestellt, mit Natriumchlorid gesättigt und dreimal mit je 30 ml Methylenchlorid extrahiert. Die organischen Extrakte werden eingedampft und der feste Rückstand durch Säulenchromatographie gereinigt (200 g Kieselgel, Elutionsmittel: Methylenchlorid mit 1 bis 3 % Ethanol).

Ausbeute: 18,6 % der Theorie,

Schmelzpunkt: 224-225°C.

$C_{15}H_{15}N_3O_3S$ (317,38)

| | | | | | | | | |
|---|---|---|---|---|---|---|---|---|
| Ber.: | C | 56,77 | H | 4,76 | N | 13,24 | S | 10,10 |
| Gef.: | | 56,23 | | 4,78 | | 12,97 | | 9,68 |

Beispiel 32

8-(2'-Methoxy-4'-methylsulfonylmethyl-phenyl)-purin

Hergestellt analog Beispiel 31 aus 8-(2'-Methoxy-4'-methylthiomethyl-phenyl)-purin und Peressigsäure.

Ausbeute: 43,6 % der Theorie,

Schmelzpunkt: 235-237°C.

$C_{14}H_{14}N_4O_3S$ (318,36)

| | | | | | | |
|---|---|---|---|---|---|---|
| Ber.: | C | 52,82 | H | 4,43 | N | 17,60 |
| Gef.: | | 52,74 | | 4,60 | | 16,81 |

Beispiel 33

2-(2'-Methoxy-4'-methylsulfinylmethyl-phenyl)-imidazo-[4,5-b]pyridin

1,4 g (4,9 mMol) 2-(2'-Methoxy-4'-methylthiomethyl-phenyl)-imidazo[4,5-b]pyridin werden in 30 ml Eisessig gelöst und mit 3 ml 30%igem Wasserstoffperoxid versetzt. Nach 2 Stunden Rühren bei Raumtemperatur wird die Lösung mit 200 ml Wasser verdünnt, mit konzentrierter Ammoniak-Lösung alkalisch gestellt, mit Natriumchlorid gesättigt und dreimal mit je 30 ml Methylenchlorid extrahiert. Die organischen Extrakte werden eingedampft und der feste Rückstand durch Säulenchromatographie gereinigt (200 g Kieselgel, Elutionsmittel: Methylenchlorid mit 2 bis 10 % Ethanol).

Ausbeute: 21,7 % der Theorie,

$C_{15}H_{15}N_3O_2S$ (301,38)

| | | | | | | |
|---|---|---|---|---|---|---|
| Ber.: | C | 59,78 | H | 5,02 | N | 10,64 |
| Gef.: | | 59,42 | | 5,54 | | 11,53 |

$^1$H-NMR-Spektrum ($CDCl_3/CD_3OD$):

= 2,6 (s,3H); 4,1 (s,5H); 6,9-7,4 (m,3H); 7,8-8,0 (m,1H); 8,2-8,4 (m,2H) ppm.

Beispiel 34

8-(2'-Methoxy-4'-methylsulfinylmethyl-phenyl)-purin

Hergestellt analog Beispiel 33 aus 8-(2'-Methoxy-4'-methylthiomethyl-phenyl)-purin und Peressigsäure.

Ausbeute: 53,7 % der Theorie,

$R_F$-Wert: 0,18 (Kieselgel, Laufmittel: Methylenchlorid/Ethanol = 9/1).

$^1$H-NMR-Spektrum ($CDCl_3/CD_3OD$):

$\delta$ = 2,6 (s,3H); 4,1 (s,5H); 7,0-7,3 (m,2H); 8,3-8,6 (m,1H); 8,8-9,1 (m,2H) ppm.

Beispiel 35

2-(2'-Methoxy-4'-cyan-phenyl)-imidazo[4,5-b]pyridin

3,1 g 2,3-Diaminopyridin und 5,0 g 2-Methoxy-4-cyan-benzoesäure werden in 50 ml Phosphoroxychlorid 2,5 Stunden zum Rückfluß erhitzt. Nach dem Abkühlen wird das Reaktionsgemisch mit Eiswasser behandelt. Der ausgefallene Niederschlag wird mit Wasser gewaschen und im Umlufttrockenschrank bei 60°C getrocknet, wonach das Produkt noch knapp ein halbes Mol Salzsäure und ein halbes Mol Wasser enthält.
Ausbeute: 6,3 g (80 % der Theorie),
Schmelzpunkt: 214-216°C (Zersetzung).
Ber. (für 0,5 Mol HCl und 0,5 Mol $H_2O$):

| | | | | | | | | |
|---|---|---|---|---|---|---|---|---|
| | C | 60,59 | H | 4,18 | N | 20,19 | Cl | 6,38 |
| Gef.: | | 60,85 | | 4,15 | | 20,48 | | 6,35 |

Beispiel 36

2-(2'-Methoxy-4'-carboxy-phenyl)-imidazo[4,5-b]pyridin

3,9 g 2-(2'-Methoxy-4'-cyan-phenyl)-imidazo[4,5-b]pyridin werden in 70 ml 2n wäßriger Natronlauge 10 Minuten unter Rühren zum Rückfluß erhitzt, wobei das Ausgangsmaterial in Lösung geht. Es wird auf etwa 50°C abgekühlt und über Aktivkohle filtriert. Nach Abkühlen auf Raumtemperatur wird mit Eisessig angesäuert und der erhaltene Niederschlag abgesaugt und mit Wasser gewaschen. Man kristallisiert schließlich aus Ethylenglykol unter Zusatz von Aktivkohle um und wäscht die ausgefallenen Kristalle mit Äthanol und Aceton.
Ausbeute: 2,3 g (55 % der Theorie),
Schmelzpunkt: 309-310°C.

| | | | | | | |
|---|---|---|---|---|---|---|
| Ber.: | C | 62,45 | H | 4,12 | N | 15,61 |
| Gef.: | | 62,30 | | 4,47 | | 15,60 |

## Beispiel 37

**2-(2'-Methoxy-4'-methoxycarbonyl-phenyl)-imidazo[4,5-b]-pyridin-hydrochlorid**

3,0 g 2-(2'-Methoxy-4'-carboxy-phenyl)-imidazo[4,5-b]pyridin werden mit 40 ml Thionylchlorid 3 Stunden zum Rückfluß erhitzt und dann im Vakuum zur Trockne eingedampft. Der Rückstand wird in 200 ml Methanol aufgekocht, die Lösung über Aktivkohle filtriert und der nach dem Abkühlen ausgefallene Niederschlag abgesaugt und mit Methanol und Ether gewaschen.
Ausbeute: 2,4 g (68 % der Theorie),
Schmelzpunkt: 238-239°C (Zersetzung).

| | | | | | | | | |
|---|---|---|---|---|---|---|---|---|
| Ber.: | C | 56,34 | H | 4,41 | N | 13,14 | Cl | 11,09 |
| Gef.: | | 55,96 | | 4,50 | | 13,30 | | 11,75 |

## Beispiel 38

**2-(2'-Methoxy-4'-aminocarbonyl-phenyl)-imidazo[4,5-b]-pyridin-hydrochlorid**

3,0 g des aus 2-(2'-Methoxy-4'-carboxy-phenyl)-imidazo-[4,5-b]pyridin und Thionylchlorid durch 1,5-stündiges Kochen und Eindampfen zur Trockne gewonnen Säurechlorid-hydrochlorid werden in 70 ml Dioxan suspendiert und dazu langsam 10 ml einer konzentrierten wäßrigen Ammoniaklösung getropft. Man rührt noch 30 Minuten bei 80°C nach, destilliert das Dioxan weitgehend ab, verrührt den Rückstand mit Wasser und saugt das ausgefallene Produkt ab. Es wird aus einer Mischung aus 120 ml Ethanol und 120 ml 2n Salzsäure umkristallisiert.
Ausbeute: 1,7 g (60 % der Theorie),
Schmelzpunkt: > 280°C.

| | | | | | | | | |
|---|---|---|---|---|---|---|---|---|
| Ber.: | C | 55,18 | H | 4,30 | N | 18,39 | Cl | 11,63 |
| Gef.: | | 55,36 | | 4,46 | | 18,29 | | 11,76 |

Beispiel 39

2-(2'-Methoxy-4'-methylaminocarbonyl-phenyl)-imidazo-[4,5-b]pyridin

Hergestellt analog Beispiel 38 aus dem entsprechenden Säurechlorid-hydrochlorid und Methylamin.
Ausbeute: 54 % der Theorie,
Schmelzpunkt: 263-265°C (aus Ethanol).

| | | | | | | |
|---|---|---|---|---|---|---|
| Ber.: | C | 63,82 | H | 5,00 | N | 19,85 |
| Gef.: | | 63,50 | | 5,38 | | 19,59 |

Beispiel 40

2-(2'-Methoxy-4'-dimethylaminocarbonyl-phenyl)-imidazo-[4,5-b]pyridin-hydrochlorid

Hergestellt analog Beispiel 38 aus dem entsprechenden Säurechlorid-hydrochlorid und Dimethylamin. Das Hydrochlorid wird aus Aceton mit etherischer Salzsäure gefällt und aus Ethanol/Essigester umkristallisiert.
Ausbeute: 52 % der Theorie,
Schmelzpunkt: 232°C (Zers.).

| | | | | | | | | |
|---|---|---|---|---|---|---|---|---|
| Ber.: | C | 57,75 | H | 5,15 | N | 16,84 | Cl | 10,85 |
| Gef.: | | 57,50 | | 5,46 | | 16,65 | | 10,94 |

Beispiel 41

8-(2'-Methoxy-4'-cyan-phenyl)-purin

Hergestellt analog Beispiel 35 aus 4,5-Diaminopyrimidin (Kristallisat aus Dihydrochlorid mit 1 Mol Kochsalz) und 2-Methoxy-4-cyan-benzoesäure.
Ausbeute: 0,7 g (20 % der Theorie),
Schmelzpunkt: 271-272°C (aus Methanol).

| | | | | | | |
|---|---|---|---|---|---|---|
| Ber.: | C | 62,14 | H | 3,61 | N | 27,88 |
| Gef.: | | 62,34 | | 3,69 | | 27,62 |

Beispiel 42

8-(2'-Methoxy-4'-carboxy-phenyl)-purin

Hergestellt durch vierstündiges Rückflußkochen einer Lösung von 1,45 g 8-(2'-Methoxy-4'-cyan-phenyl)-purin in 100 ml 25%iger Natronlauge. Die Aufarbeitung erfolgte analog Beispiel 36 jedoch ohne Umkristallisation.
Ausbeute: 1,3 g (96 % der Theorie),
Schmelzpunkt: > 250°C

| | | | | | | |
|---|---|---|---|---|---|---|
| Ber.: | C | 57,78 | H | 3,73 | N | 20,73 |
| Gef.: | | 57,40 | | 3,85 | | 20,84 |

Beispiel 43

8-(2'-Methoxy-4'-aminocarbonyl-phenyl)-purin

2,0 g 8-(2'-Methoxy-4'-carboxy-phenyl)-purin werden mit 100 ml Thionylchlorid 3 Stunden zum Rückfluß erhitzt. Überschüssiges Thionylchlorid wird abdestilliert. Dann wird dreimal mit Methylenchlorid nachgedampft. Das verbleibende Säurechlorid-hydrochlorid wird in 125 ml Dioxan suspendiert und unter Rühren 10 ml konzentrierte wäßrige Ammoniaklösung zugetropft. Die Suspension wird 1 Stunde auf dem Dampfbad erhitzt, wobei eine klare Lösung entsteht. Das Lösungsmittel wird abgedampft, der kristalline Rückstand mit Wasser verrieben, abgesaugt, mit warmer wäßriger Natriumcarbonatlösung behandelt und mit Wasser gewaschen und getrocknet.
Ausbeute: 0,53 g (26 % der Theorie),
Schmelzpunkt: > 250°C.

| | | | | | | |
|---|---|---|---|---|---|---|
| Ber.: | C | 56,86 | H | 4,50 | N | 25,85 |
| Gef.: | | 57,15 | | 4,25 | | 25,61 |

Beispiel 44

8-(2'-Methoxy-4'-methylaminocarbonyl-phenyl)-purin

Zu 0,42 g 8-(2'-Methoxy-4'-chlorcarbonyl-phenyl)-purin-hydrochlorid (siehe Beispiel 43) werden unter Kühlung 40 ml ethanolische Methylaminlösung gegeben und anschließend 30 Minuten zum Rückfluß erhitzt. Danach wird eingedampft und das Produkt durch Säulenchromatographie an Kieselgel (Elutionsmittel: Methylenchlorid/Ethanol = 8:2) gereinigt.
Ausbeute: 0,19 g (45 % der Theorie),
Schmelzpunkt: > 250°C.

| | | | | | | |
|---|---|---|---|---|---|---|
| Ber.: | C | 59,36 | H | 4,63 | N | 24,72 |
| Gef.: | | 58,98 | | 4,66 | | 24,55 |

Beispiel 45

8-(2'-Methoxy-4'-ethoxycarbonyl-phenyl)-purin

0,42 g 8-(2'-Methoxy-4'-chlorcarbonyl-phenyl)-purin-hydrochlorid (siehe Beispiel 43) werden in 40 ml Ethanol 45 Minuten zum Rückfluß erhitzt. Das Lösungsmittel wird abgedampft und der Rückstand über eine Kieselgelsäule gereinigt (Elutionsmittel: Methylenchlorid/Ethanol = 50:1 bis 19:1).
Ausbeute: 0,18 g (40 % der Theorie),
Schmelzpunkt: 210-212°C.

| | | | | | | |
|---|---|---|---|---|---|---|
| Ber.: | C | 60,40 | H | 4,73 | N | 18,78 |
| Gef.: | | 60,11 | | 4,82 | | 18,87 |

Beispiel 46

8-(2'-Methoxy-4'-aminocarbonyl-phenyl)-purin

1,0 g 8-(2'-Methoxy-4'-cyan-phenyl)-purin werden in 75 ml 2n Natronlauge gelöst und 15 Minuten zum Rückfluß erhitzt. Danach wird mit Salzsäure angesäuert und das hierbei ausgefallene Gemisch über Kieselgel (Elutionsmittel: Methylenchlorid:Ethanol = 7:3 bis 1:1) in die Komponenten getrennt.
Ausbeute: 0,1 g (10 % der Theorie),
Schmelzpunkt: > 250°C.
Darüber hinaus werden 0,4 g (39 % der Theorie) der entsprechenden Carbonsäure erhalten.

## Beispiel 47

2-(2'-Methoxy-4'-dimethylaminosulfonyl-phenyl)-imidazo-[4,5-b]pyridin-hydrochlorid

Das aus 2,5 g 2-Methoxy-4-chlorsulfonyl-benzoesäure nach Beispiel C hergestellte rohe 2-(2'-Methoxy-4'-chlorsulfonyl-phenyl)-imidazo[4,5-b]pyridin-hydrochlorid wird bei 10°C unter Rühren in 150 ml einer gesättigten wäßrigen Dimethylaminlösung eingetragen, wobei langsam ein heller Niederschlag ausfällt. Nach 20 Stunden Rühren bei Raumtemperatur wird mit 200 ml Eiswasser versetzt. Der Niederschlag wird nach dem Trocknen in 60 ml Methanol aufgenommen und 10 ml methanolische Salzsäure zugesetzt. Nach Filtration über Aktivkohle wird das Lösungsmittel abgezogen und der verbleibende Rückstand mit Aceton und Ether digeriert, abgesaugt und mit Äther gewaschen.
Ausbeute: 0,55 g (15 % der Theorie),
Schmelzpunkt: 205-210°C.

| | | | | | | | | | | |
|---|---|---|---|---|---|---|---|---|---|---|
| Ber.: | C | 48,84 | H | 4,65 | N | 15,19 | Cl | 9,16 | S | 8,69 |
| Gef.: | | 48,56 | | 4,53 | | 15,09 | | 9,44 | | 8,69 |

## Beispiel 48

2-(2'-Methoxy-4'-methylaminosulfonyl-phenyl)-imidazo[4,5-b]-pyridin-hydrochlorid

Hergestellt analog Beispiel 47 aus 2-(2'-Methoxy-4'-chlorsulfonyl-phenyl)-imidazo[4,5-b]pyridin-hydrochlorid und

40%iger wäßriger Methylaminlösung. Nach Versetzen mit Eiswasser wird mit Essigester extrahiert, eingedampft und der Rückstand wie unter Beispiel 47 weiter verarbeitet, wobei ein Halbhydrat erhalten wird.
Ausbeute: 19 % der Theorie,
Schmelzpunkt: 205-207°C (Zers.).

| | | | | | | | | | | |
|---|---|---|---|---|---|---|---|---|---|---|
| Ber.: | C | 46,22 | H | 4,73 | N | 15,40 | Cl | 9,75 | S | 8,81 |
| Gef.: | | 46,19 | | 4,86 | | 15,00 | | 10,08 | | 8,52 |

Aus den beim Waschen der Essigesterphasen anfallenden wäßrigen Phasen fiel beim Stehen über Nacht eine weitere Fraktion des Produktes als freie Base an.
Ausbeute: 10 % der Theorie,
Schmelzpunkt: 246-247°C (Zers.).

| | | | | | | | | |
|---|---|---|---|---|---|---|---|---|
| Ber.: | C | 52,81 | H | 4,43 | N | 17,60 | S | 10,07 |
| Gef.: | | 52,92 | | 4,43 | | 17,48 | | 10,27 |

## Beispiel 49

### 2-(2'-Methoxy-4'-aminosulfonyl-phenyl)-imidazo[4,5-b]pyridin-hydrochlorid

Hergestellt analog Beispiel 47 ausgehend von 2-Methoxy-4-chlorsulfonyl-benzoesäure, Ammoniak und 2,3-Diamino-pyridin.
Ausbeute: 16,4 % der Theorie,
Schmelzpunkt: 225°C.

| | | | | | | | | | | |
|---|---|---|---|---|---|---|---|---|---|---|
| Ber.: | C | 45,82 | H | 3,85 | N | 16,44 | Cl | 10,40 | S | 9,41 |
| Gef.: | | 45,67 | | 4,11 | | 16,24 | | 10,15 | | 9,18 |

## Beispiel 50

### 8-(2'-Methoxy-4'-aminosulfonyl-phenyl)-purin

Hergestellt analog Beispiel 47 aus 2-Methoxy-4-chlorsulfonyl-benzoesäure, Ammoniak und 4,5-Diaminopyrimidin (Kristallisat aus dem Dihydrochlorid mit einem Mol Kochsalz). Nach der Umsetzung mit wäßrigem Ammoniak wird die Reaktionslösung auf etwa ein Drittel eingeengt, wonach die

freie Base kristallin ausfällt.
Ausbeute: 65 % der Theorie,
Schmelzpunkt: 270°C (Zers.).

| | | | | | | | | |
|---|---|---|---|---|---|---|---|---|
| Ber.: | C | 47,21 | H | 3,63 | N | 22,94 | S | 10,50 |
| Gef.: | | 46,95 | | 3,68 | | 22,84 | | 10,50 |

Beispiel 51

8-(2'-Methoxy-4'-methylaminosulfonyl-phenyl)-purin-hydrochlorid

Hergestellt analog Beispiel 50 aus 2-Methoxy-4-chlorsulfonyl-benzoesäure, wässriger Methylamin-Lösung und 4,5-Diamino-pyrimidin. Die rohe freie Base wird mit 2n Salzsäure in das Hydrochlorid überführt und dieses durch Aufkochen mit Methanol gereinigt.
Ausbeute: 15 % der Theorie,
Schmelzpunkt: 243°C (Zers.)

| | | | | | | | | | | |
|---|---|---|---|---|---|---|---|---|---|---|
| Ber.: | C | 43,88 | H | 3,97 | N | 19,68 | Cl | 9,97 | S | 9,01 |
| Gef.: | | 43,96 | | 4,04 | | 19,67 | | 9,86 | | 8,98 |

Beispiel 52

8-(2'-Methoxy-4'-dimethylaminosulfonyl-phenyl)-purin-hydrochlorid

Hergestellt analog Beispiel 51 aus 2-Methoxy-4-chlorsulfonyl-benzoesäure, wässriger Dimethylamin-Lösung und 4,5-Diamino-pyrimidin. Das Hydrochlorid wird aus Ethanol/Wasser = 4:1 umkristallisiert.
Ausbeute: 27 % der Theorie,
Schmelzpunkt: 230-234°C.

| | | | | | | | | | | |
|---|---|---|---|---|---|---|---|---|---|---|
| Ber.: | C | 45,47 | H | 4,36 | N | 18,94 | Cl | 9,60 | S | 8,70 |
| Gef.: | | 45,11 | | 4,66 | | 19,26 | | 9,24 | | 8,43 |

Beispiel 53

8-[2'-Methoxy-4'-(4-morpholinyl-sulfonyl)-phenyl]-purin

0,8 ml Morpholin werden in einer zweiphasigen Mischung aus 40 ml Essigester und 40 ml Wasser gelöst. Dazu wird 1 g rohes 8-(2'-Methoxy-4'-chlorsulfonyl-phenyl)-purin-hydrochlorid portionsweise unter kräftigem Rühren zugegeben und anschließend 2 Stunden auf 80°C erwärmt. Der Ansatz wird auf ein Drittel des Volumens eingeengt und die ausgefallenen Kristalle abgesaugt und mit Wasser gewaschen.
Ausbeute: 0,3 g (30 % der Theorie),
Schmelzpunkt: > 250°C.

| | | | | | | | | |
|---|---|---|---|---|---|---|---|---|
| Ber.: | C | 51,20 | H | 4,56 | N | 18,66 | S | 8,54 |
| Gef.: | | 51,00 | | 4,56 | | 18,40 | | 8,80 |

Beispiel 54

8-(2'-Methoxy-4'-n-butylaminosulfonyl-phenyl)-purin

Hergestellt analog Beispiel 51 aus n-Butylamin und 8-(2'-Methoxy-4'-chlorsulfonyl-phenyl)-purin-hydrochlorid. Nach beendeter Reaktion wird die wäßrige Phase mit Essigester extahiert. Der nach dem Eindampfen der Essigesterphasen verbleibende Rückstand wird mit Ethanol digeriert, abgesaugt und mit Ether gewaschen.
Ausbeute: 0,27 g (28 % der Theorie),
Schmelzpunkt: 212-214°C (Zers.).

| | | | | | | | | |
|---|---|---|---|---|---|---|---|---|
| Ber.: | C | 53,17 | H | 5,30 | N | 19,38 | S | 8,87 |
| Gef.: | | 53,43 | | 5,46 | | 19,00 | | 8,51 |

Beispiel 55

2-(2'-Dimethylamino-4'-nitro-phenyl)-imidazo[4,5-b]pyridin

6,3 g 2-Dimethylamino-4-nitro-benzoesäure werden mit 5,43 g 2,3-Diamino-pyridin-dihydrochlorid fein verrieben, mit 125 ml Phosphoroxychlorid versetzt und 2 Stunden zum Rückfluß er- hitzt. Danach wird das überschüssige Phosphoroxychlorid größtenteils im Vakuum abdestilliert und der Rückstand mit Eiswaser versetzt und schließlich mit Ammoniak neutralisiert. Das ausgefallene Festprodukt wird durch Säulenchromatographie an Kieselgel (Elutionsmittel: zunächst Methylenchlorid, dann Methylenchlorid/Ethanol = 50:1 bis 25:1) gereinigt.
Ausbeute: 2,2 g (26 % der Theorie),
Schmelzpunkt: 208-120°C.

| | | | | | | |
|---|---|---|---|---|---|---|
| Ber.: | C | 59,36 | H | 4,63 | N | 24,72 |
| Gef.: | | 59,40 | | 4,50 | | 25,10 |

Beispiel 56

2-(2'-Dimethylamino-4'-methansulfonylamino-phenyl)-imidazo-[4,5-b]pyridin

0,49 g 2-(2'-Dimethylamino-4'-amino-phenyl)-imidazo[4,5-b]-pyridin-dihydrochlorid werden in 10 ml Pyridin gelöst, dann 0,38 g Methansulfonylchlorid zugetropft und 2 Tage bei Raumtemperatur nachgerührt. Das Reaktionsgemisch wird auf Wasser gegossen und die erhaltene Lösung mit Essigester extrahiert. Die Essigesterphasen werden mit Kochsalzlösung gewaschen und eingedampft. Der Rückstand wird mit 2n Essigsäure verrieben, abgesaugt und mit Wasser gewaschen.
Ausbeute: 0,32 g (65 % der Theorie),

Schmelzpunkt: 265-267°C.

| | | | | | | | | |
|---|---|---|---|---|---|---|---|---|
| Ber.: | C | 54,38 | H | 5,17 | N | 21,14 | S | 9,66 |
| Gef.: | | 54,10 | | 5,08 | | 21,03 | | 9,12 |

Beispiel 57

2-(2'-Dimethylamino-4'-methansulfonyloxy-phenyl)-imidazo-[4,5-b]pyridin

Hergestellt analog Beispiel 56 aus 2-(2'-Dimethylamino-4'-hydroxy-phenyl)-imidazo[4,5-b]pyridin-dihydrochlorid und Methansulfonylchlorid. Das Produkt wird über eine Kieselgelsäule gereinigt (Elutionsmittel: zunächst Methylenchlorid, später Methylenchlorid/Ethanol = 50:1).
Ausbeute: 56 % der Theorie,
Schmelzpunkt: 197-199°C.

| | | | | | | | | |
|---|---|---|---|---|---|---|---|---|
| Ber.: | C | 54,22 | H | 4,85 | N | 16,86 | S | 9,67 |
| Gef.: | | 54,31 | | 4,89 | | 16,61 | | 9,47 |

Beispiel 58

5-Acetamino-2-(2'-methoxy-4'-methansulfonyloxy-phenyl)-benzimidazol

Hergestellt analog Beispiel 1 aus 5-Acetamino-2-(2'-methoxy-4'-hydroxy-phenyl)-benzimidazol und Methansulfonsäurechlorid.
Ausbeute: 46 % der Theorie,
Schmelzpunkt: 220-222°C,
$C_{17}H_{17}N_3O_3S$ (375,41)

| | | | | | | | | |
|---|---|---|---|---|---|---|---|---|
| Ber.: | C | 54,39 | H | 4,56 | N | 11,19 | S | 8,54 |
| Gef.: | | 54,20 | | 4,50 | | 11,07 | | 8,40 |

Beispiel 59

5,6-Dimethyl-2-(2'-methoxy-4'-methansulfonyloxy-phenyl)-benzimidazol

Hergestellt analog Beispiel 1 aus 5,6-Dimethyl-2-(2'-methoxy-4'-hydroxy-phenyl)-benzimidazol und Methansulfonsäurechlorid.

Ausbeute: 39 % der Theorie,
Schmelzpunkt: 175-176°C.
$C_{17}H_{18}N_2O_4S \cdot H_2O$ (364,43)

| | | | | | | |
|---|---|---|---|---|---|---|
| Ber.: | C | 56,02 | H | 5,53 | N | 7,69 |
| Gef.: | | 56,02 | | 5,80 | | 6,98 |

Beispiel 60

2-(2'-Methoxy-4'-methansulfonyloxy-phenyl)-imidazo[4,5-c]-pyridin

Hergestellt analog Beispiel 1 aus 2-(2'-Methoxy-4'-hydroxy-phenyl)-imidazo[4,5-c]pyridin und Methansulfonsäurechlorid.

Ausbeute: 66,3 % der Theorie,
Schmelzpunkt: 208-210°C.
$C_{14}H_{13}N_3O_4S$ (319,35)

| | | | | | | |
|---|---|---|---|---|---|---|
| Ber.: | C | 52,66 | H | 4,10 | N | 13,16 |
| Gef.: | | 52,60 | | 4,21 | | 13,10 |

Beispiel 61

5-Methoxycarbonyl-2-(2'-methoxy-4'-methansulfonyloxy-phenyl)-benzimidazol

Hergestellt analog Beispiel 1 aus 5-Methoxycarbonyl-2-(2'-methoxy-4'-hydroxy-phenyl)-benzimidazol und

Methansulfonsäurechlorid.
Ausbeute: 35 % der Theorie,
Schmelzpunkt: 121-123°C.
$C_{17}H_{16}N_2O_6S \times H_2O$ (394,42)

| | | | | | | |
|---|---|---|---|---|---|---|
| Ber.: | C | 51,76 | H | 4,60 | N | 7,10 |
| Gef.: | | 52,03 | | 4,56 | | 7,14 |

Beispiel 62

5-Methansulfonylamino-2-(2'-methoxy-4'-methansulfonyloxy-phenyl)-benzimidazol

Hergestellt analog Beispiel 1 aus 5-Amino-2-(2'-methoxy-4'-hydroxy-phenyl)-benzimidazol und Methansulfonsäurechlorid.
Ausbeute: 6 % der Theorie,
Schmelzpunkt: 240°C (Zersetzung).
$C_{16}H_{17}N_3O_6S_2$ (411,47)

| | | | | | | |
|---|---|---|---|---|---|---|
| Ber.: | C | 46,70 | H | 4,16 | N | 10,21 |
| Gef.: | | 46,63 | | 4,25 | | 10,16 |

Beispiel 63

5-Methoxycarbonylamino-2-(2'-methoxy-4'-methansulfonyloxy-phenyl)-benzimidazol

Hergestellt analog Beispiel 1 aus 5-Methoxycarbonylamino-2-(2'-methoxy-4'-hydroxy-phenyl)-benzimidazol und Methansulfonsäurechlorid.
Ausbeute: 37,3 % der Theorie,
Schmelzpunkt: 140°C (Zersetzung).
$C_{17}H_{17}N_3O_6S \times H_2O$ (409,42)

| | | | | | | | | |
|---|---|---|---|---|---|---|---|---|
| Ber.: | C | 49,87 | H | 4,67 | N | 10,26 | S | 7,83 |
| Gef.: | | 50,32 | | 4,70 | | 10,49 | | 7,85 |

## Beispiel 64

### 5-Methyl-2-(2'-methoxy-4'-methansulfonyloxy-phenyl)-benzimidazol

Hergestellt analog Beispiel 1 aus 5-Methyl-2-(2'-methoxy-4'-hydroxy-phenyl)-benzimidazol.
Ausbeute: 30,6 % der Theorie,
Schmelzpunkt: 130-133°C.
$C_{16}H_{16}N_2O_4S$ (332,4)

| | | | | | | | | |
|---|---|---|---|---|---|---|---|---|
| Ber.: | C | 57,81 | H | 4,85 | N | 8,43 | S | 9,65 |
| Gef.: | | 57,66 | | 5,04 | | 8,40 | | 9,54 |

## Beispiel 65

### 5-Fluor-2-(2'-methoxy-4'-methansulfonyloxy-phenyl)-benzimidazol

Hergestellt analog Beispiel 1 aus 5-Fluor-2-(2'-methoxy-4'-hydroxy-phenyl)-benzimidazol und Methansulfonsäurechlorid.
Ausbeute: 71 % der Theorie,
Schmelzpunkt: 203-204°C.
$C_{15}H_{13}FN_2O_4S$ (336,35)

| | | | | | | | | |
|---|---|---|---|---|---|---|---|---|
| Ber.: | C | 53,56 | H | 3,90 | N | 8,33 | S | 9,53 |
| Gef.: | | 53,40 | | 3,97 | | 8,75 | | 9,61 |

## Beispiel 66

### 2-(2'-Methoxy-4'-trifluormethansulfonyloxy-phenyl)-benzimidazol

Hergestellt analog Beispiel 1 aus 2-(2'-Methoxy-4'-hydroxy-

phenyl)-benzimidazol und Trifluormethansulfonsäureanhydrid.
Ausbeute: 44,6 % der Therorie,
Schmelzpunkt: 191-193°C.
$C_{15}H_{11}F_3N_2O_4S$ (372,3)

| | | | | | | | | |
|---|---|---|---|---|---|---|---|---|
| Ber.: | C | 48,39 | H | 2,98 | N | 7,53 | S | 8,61 |
| Gef.: | | 48,08 | | 3,20 | | 7,48 | | 9,06 |

Beispiel 67

2-(2'-Methoxy-4'-trifluormethansulfonyloxy-phenyl)-imidazo-[4,5-b]pyridin

Hergestellt analog Beispiel 1 aus 2-(2'-Methoxy-4'-hydroxy-phenyl)-imidazo[4,5-b]pyridin und Trifluormethansulfonsäureanhydrid.
Ausbeute: 53,8 % der Theorie,
Schmelzpunkt: 205-207°C.
$C_{14}H_{10}F_3N_3O_4S$ (373.3)

| | | | | | | |
|---|---|---|---|---|---|---|
| Ber.: | C | 45,05 | H | 2,70 | N | 11,26 |
| Gef.: | | 45,29 | | 2,75 | | 11,38 |

Beispiel 68

8-(2'-n-Propyl-4'-methansulfonyloxy-phenyl)-purin

Hergestellt analog Beispiel 1 aus 8-(2'-n-Propyl-4'-hydroxy-phenyl)-purin und Methansulfonsäurechlorid.
Ausbeute: 64,1 % der Theorie,
Schmelzpunkt: 214-216°C.
$C_{15}H_{16}N_4O_3S$ (332,4)

| | | | | | | |
|---|---|---|---|---|---|---|
| Ber.: | C | 54,20 | H | 4,85 | N | 16,86 |
| Gef.: | | 54,45 | | 4,77 | | 17,00 |

Beispiel 69

8-(2'-Methoxy-4'-trifluormethansulfonyloxy-phenyl)-purin

Hergestellt analog Beispiel 1 aus 8-(2'-Methoxy-4'-hydroxy-phenyl)-purin und Trifluormethansulfonsäureanhydrid.
Ausbeute: 37,4 % der Theorie,
Schmelzpunkt: 228-229°C.

$C_{13}H_9F_3N_4O_4S$ (374,3)

| | | | | | | |
|---|---|---|---|---|---|---|
| Ber.: | C | 41,72 | H | 2,42 | N | 14,97 |
| Gef.: | | 41,75 | | 2,50 | | 15,20 |

Beispiel 70

8-(2'-Ethyl-4'-methansulfonyloxy-phenyl)-purin

Hergestellt analog Beispiel 1 aus 8-(2'-Ethyl-4'-hydroxy-phenyl)-purin und Methansulfonsäurechlorid.
Ausbeute: 69,2 % der Theorie,
Schmelzpunkt: 237-238°C.

$C_{14}H_{14}N_4O_3S$ (318.4)

| | | | | | | |
|---|---|---|---|---|---|---|
| Ber.: | C | 52,81 | H | 4,43 | N | 17.60 |
| Gef.: | | 53,00 | | 4,39 | | 17,70 |

Beispiel 71

6-Methyl-2-(2'-methoxy-4'-methansulfonyloxy-phenyl)-imidazo-[4,5-b]pyridin

Hergestellt analog Beispiel 1 aus 6-Methyl-2-(2'-methoxy-4'-hydroxy-phenyl)-imidazo[4,5-b]pyridin und Methansulfonsäurechlorid.
Ausbeute: 38,6 % der Theorie,
Schmelzpunkt: 185-187°C.
$C_{15}H_{15}N_3O_4S$ (333.4)

| | | | | | | | | |
|---|---|---|---|---|---|---|---|---|
| Ber.: | C | 54.04 | H | 4,54 | N | 12,60 | S | 9,62 |
| Gef.: | | 54,04 | | 4,55 | | 12,68 | | 9,50 |

Beispiel 72

5-Nitro-2-(2'-methoxy-4'-N-methyl-methansulfonylamino-phenyl)-benzimidazol-hydrochlorid

Hergestellt analog Beispiel 14 aus 4-Nitro-1,2-phenylendiamin und 2-Methoxy-4-N-methyl-methansulfonylamino-benzoesäure.
Ausbeute: 52,1 % der Theorie,
Schmelzpunkt: 241-243°C.
$C_{16}H_{16}N_4O_5S$ x HCl (412,87)

| | | | | | | | | | | |
|---|---|---|---|---|---|---|---|---|---|---|
| Ber.: | C | 46,55 | H | 4,15 | N | 13,56 | S | 7,77 | Cl | 8,58 |
| Gef.: | | 46,75 | | 3,94 | | 13,68 | | 7,72 | | 8,29 |

Beispiel 73

5-Methoxy-2-(2'-methoxy-4'-N-methyl-methansulfonylamino-phenyl)-benzimidazol

Hergestellt analog Beispiel 14 aus 4-Methoxy-1,2-phenylendiamin und 2-Methoxy-4-N-methyl-methansulfonylamino-benzoesäure.
Ausbeute: 33,7 % der Theorie,
Schmelzpunkt: 194-196°C.
$C_{17}H_{19}N_3O_4S$ (361,43)

| | | | | | | | | |
|---|---|---|---|---|---|---|---|---|
| Ber.: | C | 56,49 | H | 5,30 | N | 11,63 | S | 8,87 |
| Gef.: | | 56,49 | | 5,40 | | 11,73 | | 8,84 |

Beispiel 74

5-Trifluormethyl-2-(2'-methoxy-4'-N-methyl-methansulfonylamino-phenyl)-benzimidazol

Hergestellt analog Beispiel 14 aus 4-Trifluormethyl-1,2-phenylendiamin und 2-Methoxy-4-N-methyl-methansulfonylamino-benzoesäure.
Ausbeute: 6,7 % der Theorie
Schmelzpunkt: 222-225°C.
$C_{17}H_{16}F_3N_3O_3S$ (399,41)

| | | | | | | | | |
|---|---|---|---|---|---|---|---|---|
| Ber.: | C | 51,12 | H | 4,04 | N | 10,52 | S | 8,02 |
| Gef.: | | 51,34 | | 4,38 | | 10,28 | | 8,47 |

Beispiel 75

5-Trifluormethyl-2-(2'-methoxy-4'-methansulfonylamino-phenyl)-benzimidazol

Hergestellt analog Beispiel 14 aus 4-Trifluormethyl-1,2-phenylendiamin und 2-Methoxy-4-methansulfonylamino-benzoesäure.
Ausbeute: 24,9 % der Theorie,
Schmelzpunkt: 115-118°C.
$C_{16}H_{14}F_3N_3O_3S$ (385,33)

| | | | | | | | | |
|---|---|---|---|---|---|---|---|---|
| Ber.: | C | 49,87 | H | 3,66 | N | 10,90 | S | 8,32 |
| Gef.: | | 49,64 | | 3,89 | | 10,65 | | 8,34 |

Beispiel 76

5-Nitro-2-(2'-methoxy-4'-methansulfonylamino-phenyl)-benzimidazol-dihydrochlorid

Hergestellt analog Beispiel 14 aus 4-Nitro-1,2-phenylendiamin und 2-Methoxy-4-methansulfonylamino-benzoesäure.
Ausbeute: 14,0 % der Theorie,

Schmelzpunkt: 240-243°C.

$C_{15}H_{14}N_4O_5S \times H_2O \times 2\ HCl$ (453,33)

| | | | | | | |
|---|---|---|---|---|---|---|
| Ber.: | C | 39,74 | H | 4,00 | N | 12,36 |
| Gef.: | | 39,56 | | 4,06 | | 12,40 |

## Beispiel 77

**5,6-Dimethyl-2-(2'-methoxy-4'-N-methyl-methansulfonylamino-phenyl)-benzimidazol**

Hergestellt analog Beispiel 14 aus 4,5-Dimethyl-1,2-phenylendiamin und 2-Methoxy-4-N-methyl-methansulfonylamino-benzoesäure.

Ausbeute: 52,3 % der Theorie,

Schmelzpunkt: 235-238°C.

$C_{18}H_{21}N_3O_3S$ (359,46)

| | | | | | | | | |
|---|---|---|---|---|---|---|---|---|
| Ber.: | C | 60,14 | H | 5,88 | N | 11,69 | S | 8,92 |
| Gef.: | | 59,80 | | 5,68 | | 11,75 | | 8,86 |

## Beispiel 78

**5,6-Dimethyl-2-(2'-methoxy-4'-methansulfonylamino-phenyl)-benzimidazol-semihydrochlorid**

Hergestellt analog Beispiel 14 aus 4,5-Dimethyl-1,2-phenylendiamin und 2-Methoxy-4-methansulfonylamino-benzoesäure.

Ausbeute: 25,6 % der Theorie,

Schmelzpunkt: 148-151°C.

$C_{17}H_{19}N_3O_3S \times 1/2\ HCl$ (363,67)

| | | | | | | |
|---|---|---|---|---|---|---|
| Ber.: | C | 56,14 | H | 5,54 | N | 11,55 |
| Gef.: | | 56,26 | | 5,76 | | 11,68 |

Beispiel 79

5,6-Dimethoxy-2-(2'-methoxy-4'-N-methyl-methansulfonylamino-phenyl)-benzimidazol-hydrochlorid

Hergestellt analog Beispiel 14 aus 4,5-Dimethoxy-1,2-phenylendiamin und 2-Methoxy-4-N-methyl-methansulfonylamino-benzoesäure.
Ausbeute: 21 % der Theorie,
Schmelzpunkt: > 250°C.
$C_{18}H_{21}N_3O_5S$ x HCl (427,93)

| | | | | | | |
|---|---|---|---|---|---|---|
| Ber.: | C | 50,52 | H | 5,18 | N | 9,82 |
| Gef.: | | 50,30 | | 5,10 | | 9,89 |

Beispiel 80

5,6-Dimethoxy-2-(2'-methoxy-4'-methansulfonylamino-phenyl)-benzimidazol

Hergestellt analog Beispiel 14 aus 4,5-Dimethoxy-1,2-phenylendiamin und 2-Methoxy-4-methansulfonylamino-benzoesäure.
Ausbeute: 27,8 % der Theorie,
Schmelzpunkt: > 250°C.
$C_{17}H_{19}N_3O_5S$ (377,49)

| | | | | | | |
|---|---|---|---|---|---|---|
| Ber.: | C | 54,09 | H | 5,07 | N | 11,13 |
| Gef.: | | 53,84 | | 5,32 | | 10,78 |

Beispiel 81

5-Methoxycarbonyl-2-(2'-methoxy-4'-methansulfonylamino-phenyl-benzimidazol

Hergestellt analog Beispiel 14 aus 4-Methoxycarbonyl-1,2-phenylendiamin und 2-Methoxy-4-methansulfonylamino-benzoesäure.

Ausbeute: 46,3 % der Theorie,

Schmelzpunkt: 246-248°C.

$C_{17}H_{17}N_3O_5S$ (375,41)

| | | | | | | |
|---|---|---|---|---|---|---|
| Ber.: | C | 54,39 | H | 4,56 | N | 11,19 |
| Gef.: | | 53,98 | | 4,72 | | 10,93 |

Beispiel 82

5-Fluor-2-(2'-methoxy-4'-methansulfonylamino-phenyl)-benzimidazol

Hergestellt analog Beispiel 14 aus 4-Fluor-1,2-phenylendiamin und 2-Methoxy-4-methansulfonylamino-benzoesäure.

Ausbeute: 67,3 % der Theorie,

Schmelzpunkt: 254-256°C.

$C_{15}H_{14}FN_3O_3S$ (335,37)

| | | | | | | | | |
|---|---|---|---|---|---|---|---|---|
| Ber.: | C | 53,72 | H | 4,20 | N | 12,53 | S | 9,56 |
| Gef.: | | 53,83 | | 4,87 | | 12,06 | | 9,25 |

Beispiel 83

6-Chlor-2-(2'-methoxy-4'-methansulfonylamino-phenyl)-imidazo-[4,5-b]pyridin-hydrochlorid

Hergestellt analog Beispiel 14 aus 5-Chlor-2,3-diaminopyridin und 2-Methoxy-4-methansulfonylamino-benzoesäure.

Ausbeute: 28,3 % der Theorie,

Schmelzpunkt: > 250°C.

$C_{14}H_{13}ClN_4O_3S \times HCl$ (389,3)

| | | | | | | |
|---|---|---|---|---|---|---|
| Ber.: | C | 43,19 | H | 3,62 | N | 14,39 |
| Gef.: | | 43,34 | | 4,05 | | 14,80 |

Beispiel 84

6-Methyl-2-(2'-methoxy-4'-methansulfonylamino-phenyl)-imidazo[4,5-b]pyridin

Hergestellt analog Beispiel 14 aus 5-Methyl-2,3-diaminopyridin und 2-Methoxy-4-methansulfonylamino-benzoesäure.
Ausbeute: 42,2 % der Theorie,
Schmelzpunkt: 253-256°C.

$C_{15}H_{16}N_4O_3S$ (332,4)

| | | | | | | |
|---|---|---|---|---|---|---|
| Ber.: | C | 54,20 | H | 4,85 | N | 16,86 |
| Gef.: | | 53,91 | | 4,97 | | 16,51 |

$^1$H-NMR-Spektrum (DMSO-$d_6$/$CD_3OD$):
$\delta$ = 2,5 (s,3H); 3,2 (s,3H); 4,1 (s,3H); 6,9-7,2 (m,2H); 7,8 (breites s,1H); 8,2-8,4 (m,2H) ppm.

Analog wurde folgende Verbindung hergestellt:

6-Methyl-2-(2'-methoxy-4'-N-ethyl-methansulfonylamino-phenyl)-imidazo[4,5-b]pyridin

Hergestellt aus 5-Methyl-2,3-diaminopyridin und 2-Methoxy-4-N-ethyl-methansulfonylamino-benzoesäure.
Ausbeute: 37,8 % der Theorie,

$C_{17}H_{20}N_4O_3S$ (360,4)

| | | | | | | |
|---|---|---|---|---|---|---|
| Ber.: | C | 56,65 | H | 5,59 | N | 15,55 |
| Gef.: | | 56,99 | | 5,64 | | 15,21 |

$^1$H-NMR-Spektrum (DMSO-$d_6$/$CD_3OD$):
$\delta$ = 1,0-1,3 (t,3H); 2,5 (s,3H); 3,1 (s,3H); 3,6-4,0 (q,2H); 4,1 (s,3H); 7,1-7,4 (m,2H); 7,8-7,9 (breites s,1H); 8,2-8,5 (m,2H)ppm.

Beispiel 85

6-Methyl-2-(2'-methoxy-4'-N-methyl-methansulfonylamino-phenyl)-imidazo[4,5-b]pyridin

Hergestellt analog Beispiel 14 aus 5-Methyl-2,3-diaminopyridin und 2-Methoxy-4-N-methyl-methansulfonylamino-benzoesäure.
Ausbeute: 46,2 % der Theorie,
Schmelzpunkt: 246-248°C.
$C_{16}H_{18}N_4O_3S$ (346,4)

| | | | | | | | | |
|---|---|---|---|---|---|---|---|---|
| Ber.: | C | 55,48 | H | 5,24 | N | 16,18 | S | 9,26 |
| Gef.: | | 55,26 | | 5,28 | | 16,35 | | 9,14 |

Beispiel 86

2-(2'-Methoxy-5'-methansulfonylamino-phenyl)-imidazo[4,5-b]-pyridin

Hergestellt analog Beispiel 14 aus 2,3-Diaminopyridin und 2-Methoxy-5-methansulfonylamino-benzoesäure.
Ausbeute: 14,2 % der Theorie,
Schmelzpunkt: > 250°C.
$C_{14}H_{14}N_4O_3S$ (318,4)

| | | | | | | | | |
|---|---|---|---|---|---|---|---|---|
| Ber.: | C | 52,81 | H | 4,43 | N | 17,60 | S | 10,07 |
| Gef.: | | 52,73 | | 4,63 | | 17,25 | | 10,79 |

Beispiel 87

5-Amino-2-(2'-methoxy-4'-methansulfonyloxy-phenyl)-benzimidazol

5,0 g (13,3 mMol) 5-Acetamino-2-(2'-methoxy-4'-methansulfonyloxy-phenyl)-benzimidazol werden in 150 ml konzentrierter

Salzsäure suspendiert und 2 Stunden bei 80°C gerührt. Nach dem Abkühlen wird das ausgefallene Rohprodukt abgesaugt und über 500 g Aluminiumoxid (neutral) chromatographiert (Elutionsmittel: Methylenchlorid mit 2,5 % Ethanol).
Ausbeute: 80,5 % der Theorie,
$C_{15}H_{15}N_3O_4S$ (333,38).
Massenspektrum: $M^+$ = 333 (Mol-Peak)

| | | | | | | |
|---|---|---|---|---|---|---|
| Ber.: | C | 54,04 | H | 4,54 | N | 12,61 |
| Gef.: | | 53,98 | | 4,51 | | 12,73 |

Beispiel 88

5-Hydroxy-2-(2'-methoxy-4'-methansulfonyloxy-phenyl)-benzimidazol

3,2 g (9,6 mMol) 5-Amino-2-(2'-methoxy-4'-methansulfonyloxy-phenyl)-benzimidazol werden in 509 ml Eisessig gelöst, dann unter Rühren eine Lösung von 1,38 g (20 mMol) Natriumnitrit in 5,0 ml Wasser zugetropft. Nach 1 Stunde wird mit 100 ml Wasser verdünnt und die Lösung 1 1/2 Stunden lang zum Rückfluß erhitzt. Nach dem Abkühlen wird mit konzentriertem Ammoniak auf pH 5 eingestellt, dreimal mit je 40 ml Methylethylketon extrahiert, die Extrakte getrocknet und im Vakuum eingeengt. Der ölige Rückstand wird über 500 g Aluminiumoxid (neutral) chromatographiert (Elutionsmittel: Methylenchlorid mit 8 % Ethanol).
Ausbeute: 19,6 % der Theorie,
Schmelzpunkt: 158-160°C.
$C_{15}H_{14}N_2O_5S$ (334,36)

| | | | | | | | | |
|---|---|---|---|---|---|---|---|---|
| Ber.: | C | 53,88 | H | 4,22 | N | 8,38 | S | 9,59 |
| Gef.: | | 54,05 | | 4,44 | | 8,10 | | 9,55 |

Beispiel 89

5-Methylaminocarbonylamino-2-(2'-methoxy-4'-methansulfonyloxy-phenyl)-benzimidazol

1,0 g (3,0 mMol) 5-Amino-2-(2'-methoxy-4'-methansulfonyloxy-phenyl)-benzimidazol wird in 30 ml reinem Tetrahydrofuran gelöst, 2,0 ml Methylisocyanat hinzugegeben und zum Rückfluß erhitzt. Nach 15 Minuten dampft man das Lösungsmittel und überschüssiges Methylisocyanat im Vakuum ab und der erhaltene Rückstand wird über 250 g Aluminiumoxid (neutral) chromatographiert (Elutionsmittel: Methylenchlorid mit 4 % Ethanol).

Ausbeute: 61,5 % der Theorie,

$C_{17}H_{18}N_4O_5S$ (390,43).

| | | | | | | | | |
|---|---|---|---|---|---|---|---|---|
| Ber.: | C | 52,30 | H | 4,65 | N | 14,35 | S | 8,21 |
| Gef.: | | 52,15 | | 4,83 | | 14,46 | | 7,97 |

Beispiel 90

5-Aminocarbonylamino-2-(2'-methoxy-4'-methansulfonyloxy-phenyl)-benzimidazol

1,0 g (3,0 mMol) 5-Amino-2-(2'-methoxy-4'-methansulfonyloxy-phenyl)-benzimidazol werden in 20 ml reinem Tetrahydrofuran gelöst, dann nacheinander 2,0 g Kaliumcyanat und 5 ml 2 m Essigsäure zugesetzt. Nach 8-stündigem Erhitzen unter Rückfluß dampft man das Lösungsmittel im Vakuum ab, der Rückstand wird mit 10 ml Wasser versetzt und mit 5 %iger Natriumhydrogencarbonat-Lösung neutralisiert. Das ausgefallene Rohprodukt wird abgesaugt und über 200 g Kieselgel chromatographiert (Elutionsmittel: Methylenchlorid mit 8 % Ethanol).

Ausbeute: 34,5 % der Theorie,
$C_{16}H_{16}N_4O_5S$ (376,40).

| | | | | | | | | |
|---|---|---|---|---|---|---|---|---|
| Ber.: | C | 51,06 | H | 4,18 | N | 14,54 | S | 8,32 |
| Gef.: | | 50,81 | | 4,16 | | 14,29 | | 8,71 |

## Beispiel 91

**5-Methylaminocarbonyl-2-(2'-methoxy-4'-methansulfonylamino-phenyl)-benzimidazol**

1,5 g (4,0 mMol) 5-Methoxycarbonyl-2-(2'-methoxy-4'-methansulfonylamino-phenyl)-benzimidazol und 10 ml Methylamin werden in einer Stahlbombe 4 Stunden lang auf 120°C erhitzt. Anschließend wird das überschüssige Methylamin abgedampft, der Rückstand in wenig Wasser gelöst und mit konzentrierter Salzsäure neutralisiert. Nach Eindampfen der Lösung wird das erhaltene Rohprodukt durch Chromatographie gereinigt (150 g Kieselgel, Elutionsmittel: Methylenchlorid mit 10 % Ethanol).
Ausbeute: 67,0 % der Theorie,
Schmelzpunkt: 120-124°C.
$C_{17}H_{18}N_4O_4S$ (374,43)

| | | | | | | | | |
|---|---|---|---|---|---|---|---|---|
| Ber.: | C | 54,53 | H | 4,85 | N | 14,96 | S | 8,56 |
| Gef.: | | 54,21 | | 4,97 | | 14,87 | | 8,28 |

## Beispiel 92

**5-Hydroxymethyl-2-(2'-methoxy-4'-methansulfonylamino-phenyl)-benzimidazol**

1,9 g (5,1 mMol) 5-Methoxycarbonyl-2-(2'-methoxy-4'-methansulfonylamino-phenyl)-benzimidazol, gelöst in 300 ml absolutem Tetrahydrofuran, werden unter Rühren zu einer Suspension von 700 mg Lithiumaluminiumhydrid in 50 ml absolutem Tetrahydrofuran zugetropft. Nach Rühren über Nacht bei Raumtemperatur versetzt man die Lösung mit 30 ml Wasser, filtriert von ungelösten Bestandteilen ab und engt das

Filtrat im Vakuum zur Trockne ein. Das so erhaltene Rohprodukt wird durch Chromatographie über 200 g Kieselgel (Elutionsmittel: Methylenchlorid mit 3-10 % Ethanol) gereinigt.
Ausbeute: 83,3 % der Theorie,
$C_{16}H_{17}N_3O_4S$ (347,4).

| | | | | | | | | |
|---|---|---|---|---|---|---|---|---|
| Ber.: | C | 55,32 | H | 4,93 | N | 12,10 | S | 9,23 |
| Gef.: | | 54,95 | | 5,22 | | 11,79 | | 9,14 |

Beispiel 93

5-Amino-2-(2'-methoxy-4'-N-methyl-methansulfonamino-phenyl)-benzimidazol

5,8 g (15,4 mMol) 5-Nitro-2-(2'-methoxy-4'-methylmethansulfonamino-phenyl)-benzimidazol werden in 250 ml Ethanol suspendiert, 7,5 g Raney-Nickel zugegeben und unter Rühren 20 ml Hydrazinhydrat zugetropft. Dann wird über Nacht bei Raumtemperatur gerührt, anschließend vom Katalysator abgesaugt, das Filtrat eingeengt und der Rückstand aus Ethanol umkristallisiert.
Ausbeute: 20,5 % der Theorie,
Schmelzpunkt: 216-218°C.
$C_{16}H_{18}N_4O_3S$ (346,42)

| | | | | | | | | |
|---|---|---|---|---|---|---|---|---|
| Ber.: | C | 55,47 | H | 5,24 | N | 16,17 | S | 9,26 |
| Gef.: | | 55,22 | | 5,38 | | 16,00 | | 9,24 |

Beispiel 94

5-Amino-2-(2'-methoxy-4'-methansulfonylamino-phenyl)-benzimidazol-dihydrochlorid

Hergestellt analog Beispiel 93 aus 5-Nitro-2-(2'-methoxy-4'-methansulfonylamino-phenyl)-benzimidazol mit Hydrazinhydrat und Raney-Nickel.
Ausbeute: 68,5 % der Theorie,
Schmelzpunkt: 215-217°C.

$C_{15}H_{16}N_4O_3S$ x 2 HCl (405,32)

| | | | | | | | | | | |
|---|---|---|---|---|---|---|---|---|---|---|
| Ber.: | C | 44,45 | H | 4,48 | N | 13,82 | S | 7,91 | Cl | 17,50 |
| Gef.: | | 44,08 | | 4,71 | | 13,92 | | 7,80 | | 17,75 |

Beispiel 95

5-Hydroxy-2-(2'-methoxy-4'-methansulfonylamino-phenyl)-benzimidazol

1,6 g (10 mMol) 4-Hydroxy-1,2-phenylendiamin und 4,9 g (20 mMol) 2-Methoxy-4-methansulfonylamino-benzoesäure werden unter Rühren 1,5 Stunden lang in 80 ml Phosphoroxychlorid zum Rückfluß erhitzt. Danach wird die dunkle Lösung von ungelösten Bestandteilen abdekantiert, das Phosphoroxychlorid im Vakuum abdestilliert und der Rückstand vorsichtig mit 10 g zerstoßenem Eis versetzt. Der so erhaltenen Suspension werden 10 ml 4 n Natronlauge zugesetzt und dann 1 Stunde bei Raumtemperatur gerührt. Danach neutralisiert man unter Kühlung mit konzentrierter Salzsäure, dampft die Lösung im Vakuum auf ca. 3-4 ml ein, saugt das ausgefallene Rohprodukt ab und reinigt das erhaltene Rohprodukt chromatographisch (250 g Kieselgel, Elutionsmittel: Methylenchlorid mit 5 % Ethanol).
Ausbeute: 9,7 % der Theorie,
Schmelzpunkt: Zersetzung ab 150°C.

$C_{15}H_{15}N_3O_4S$ (333,38)

| | | | | | | | | |
|---|---|---|---|---|---|---|---|---|
| Ber.: | C | 54,04 | H | 4,53 | N | 12,60 | S | 9,62 |
| Gef.: | | 54,36 | | 4,91 | | 12,31 | | 9,77 |

Beispiel 96

5-Aminocarbonyl-2-(2'-methoxy-4'-methansulfonyloxy-phenyl)-benzimidazol

1 g (2,91 mMol) 5-Cyano-2-(2'-methoxy-4'-methansulfonyloxy-phenyl)-benzimidazol wird portionsweise zu 15 ml konzentrierter Schwefelsäure gegeben und bei Raumtemperatur 24 Stunden lang gerührt. Dann wird die Lösung auf 300 ml Eiswasser gegossen, das dabei ausgefallene Produkt abgesaugt und aus Methanol umkristallisiert.
Ausbeute: 830 mg (78,9 % der Theorie),
Schmelzpunkt: sintert ab 185°C.

Beispiel 97

2-(2-Methoxy-4-methansulfonylamino-phenyl)-6-hydroxy-imidazo-[4,5-b]pyridin

Hergestellt analog Beispiel 14 aus 2,3-Diamino-5-acetoxy-pyridin und 2-Methoxy-6-methansulfonylamino-benzoesäure. Das Produkt wird über eine Kieselgelsäule (Elutionsmittel zunächst Methylenchlorid, dann Methylenchlorid/Ethanol 50:1 bis 9:1) gereinigt.
Ausbeute: 0,06 g (69 % der Theorie),
Schmelzpunkt: 225°C (Zers.)
Massenspektrum: Molmasse 334

Beispiel 98

2-(2'-Methoxy-4'-methylthiomethyl-phenyl)-imidazo[4,5-c]-pyridin

Hergestellt analog Beispiel 14 aus 3,4-Diaminopyridin und 2-Methoxy-4-methylthiomethyl-benzoesäure.
Ausbeute: 15,8 % der Theorie,
$C_{15}H_{15}N_3OS$ (285,35)

| | | | | | | |
|---|---|---|---|---|---|---|
| Ber.: | C | 63,13 | H | 5,30 | N | 14,73 |
| Gef.: | | 62,91 | | 4,99 | | 14,48 |

$^1$H-NMR-Spektrum (DMSO-$d_6$/$CD_3OD$):
$\delta$ = 2,1 (s,3H); 3,7 (s,2H); 4,1 (s,3H); 6,9-7,3 (m,2H): 8,0-8,7 (m,3H); 9,25-9,35 (breites s,1H) ppm.

Beispiel 99

2-(2'-Methoxy-4'-methylsulfinylmethyl-phenyl)-imidazo-[4,5-c]pyridin

Hergestellt analog Beispiel 33 aus 2-(2'-Methoxy-4'-methylthiomethyl-phenyl)-imidazo[4,5-c]pyridin und Peressigsäure.
Ausbeute: 16,8 % der Theorie,
$^1$H-NMR-Spektrum (DMSO-$d_6$/$CD_3OD$):
$\delta$ = 2,6 (s,3H); 4,1 (s,5H); 6,9-7,3 (m,2H); 8,0-8,7 (m,3H); 9,3-9,4 (breites s,1H) ppm.

Beispiel 100

2-(2'-Methoxy-4'-methylsulfonylmethyl-phenyl)-imidazo[4,5-c]-pyridin

Hergestellt analog Beispiel 31 aus 2-(2'-Methoxy-4'-methylthiomethyl-phenyl)-imidazo[4,5-c]pyridin und Peressigsäure.
Ausbeute: 38,5 % der Theorie,
$C_{15}H_{15}N_3O_3S$ (317,38)

| | | | | | | |
|---|---|---|---|---|---|---|
| Ber.: | C | 56,77 | H | 4,76 | N | 13,24 |
| Gef.: | | 56,41 | | 4,51 | | 12,78 |

$^1$H-NMR-Spektrum (DMSO-$d_6$/$CD_3OD$):
$\delta$ = 2,9 (s,3H); 4,1 (s,3H); 4,4 (s,2H); 7,0-7,4 (m,2H); 8,0-8,6 (m,3H); 9,2-9,3 (breites s,1H) ppm.

Beispiel 101

2-(2'-Methoxy-4'-N-methyl-trifluormethansulfonylamino-phenyl)-imidazo[4,5-b]pyridin

Hergestellt analog Beispiel 14 aus 2,3-Diaminopyridin und 2-Methoxy-4-N-methyl-trifluormethansulfonylamino-benzoesäure.
Ausbeute: 12,1 % der Theorie,
Schmelzpunkt: > 250°C.
$C_{15}H_{13}F_3N_4O_3S$ (386,35)

| | | | | | | |
|---|---|---|---|---|---|---|
| Ber.: | C | 46,63 | H | 3,39 | N | 14,50 |
| Gef.: | | 46,93 | | 3,48 | | 14,11 |

Beispiel 102

2-(2'-Methoxy-4'-N-ethyl-trifluormethansulfonylamino-phenyl)-benzimidazol-hydrochlorid

Hergestellt analog Beispiel 14 aus o-Phenylendiamin und 2-Methoxy-4-N-ethyl-trifluormethansulfonylamino-benzoesäure.
Ausbeute: 24,7 % der Theorie.
$C_{13}H_{17}ClF_3N_3O_3S$ (435,85)

| | | | | | | |
|---|---|---|---|---|---|---|
| Ber.: | C | 46,84 | H | 3,93 | N | 9,64 |
| Gef.: | | 47,12 | | 4,01 | | 9,33 |

Beispiel 103

2-(2'-Methoxy-4'-trifluormethansulfonylamino-phenyl)-benzimidazol-hydrochlorid

Hergestellt analog Beispiel 14 aus o-Phenylendiamin und 2-Methoxy-4-trifluormethansulfonylamino-benzoesäure.
Ausbeute: 18,3 % der Theorie,

Schmelzpunkt: > 220°C.

$C_{15}H_{13}ClF_3N_3O_3S$ (407,8)

| | | | | | | |
|---|---|---|---|---|---|---|
| Ber.: | C | 44,18 | H | 3,21 | N | 10,31 |
| Gef.: | | 44,33 | | 3,17 | | 10,11 |

Beispiel 104

2-(2'-Methoxy-4'-trifluormethansulfonylamino-phenyl)-imidazo-[4,5-b]pyridin-hydrochlorid

Hergestellt analog Beispiel 14 aus 2,3-Diaminopyridin und 2-Methoxy-4-trifluormethansulfonylamino-benzoesäure. Ausbeute: 15,1 % der Theorie, Schmelzpunkt: > 220°C.

$C_{14}H_{12}ClF_3N_4O_3S$ (408,8)

| | | | | | | |
|---|---|---|---|---|---|---|
| Ber.: | C | 41,13 | H | 2,96 | N | 13,71 |
| Gef.: | | 40,88 | | 2,79 | | 13,52 |

Beispiel A

Tabletten zu 100 mg 8-(2'-Methoxy-4'-methansulfonyloxy-phenyl)-purin

Zusammensetzung
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm
Dem Granulat die restlichen Hilfsstoffe zumischen und Endmischung zu Tabletten verpressen.
Tablettengewicht: 175 mg
Stempel: 8 mm

Beispiel B

Dragées zu 50 mg 8-(2'-Methoxy-4'-methansulfonyloxy-phenyl)-purin

Zusammensetzung:
1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

| | |
|---|---|
| Feuchtsiebung: | 1,0 mm |
| Trockensiebung: | 1,0 mm, |
| Trocknung: | 50°C im Umlufttrockenschrank |

Granulat und restliche Hilfsstoffe mischen und zu Kernen verpressen.

| | |
|---|---|
| Kerngewicht: | 80 mg |
| Stempel: | 6 mm |
| Wölbungsradius: | 5 mm |

Die fertigen Kerne werden auf übliche Weise mit einem Zuckerüberzug im Dragierkessel versehen.

Dragéegewicht: 120 mg

## Beispiel C

**Suppositorien zu 75 mg 8-(2'-Methoxy-4'-methansulfonyloxy-phenyl)-purin**

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

## Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 8-(2'-Methoxy-4'-methansulfonyloxy-phenyl)-imidazo/4,5-b/pyridin

1 Ampulle enthält:

| | |
|---|---|
| Wirksubstanz | 50,0 mg |
| Sorbit | 250,0 mg |
| Dest. Wasser ad | 5,0 ml |

Herstellungsverfahren:

Die Wirksubstanz und Sorbit werden in dest. Wasser gelöst, dann wird das angegebene Volumen aufgefüllt und steril filtriert.

Abfüllung: in Ampullen zu 5 ml

Sterilisation: 20 Minuten bei 120°C

Beispiel E

Tropfen zu 250 mg 8-(2'-Methoxy-4-'methansulfonyloxy-phenyl)-imidazo/4,5-b/pyridin

| | | | |
|---|---|---|---|
| Wirksubstanz | | 5,0 | g |
| p-Oxybenzoesäuremethylester | | 0,035 | g |
| p-Oxybenzoesäurepropylester | | 0,015 | g |
| Anisöl | | 0,05 | g |
| Methanol | | 0,06 | g |
| Saccharin-Natrium | | 1,0 | g |
| Glycerin | | 10,0 | g |
| Äthanol | | 40,0 | g |
| Dest. Wasser | ad | 100,0 | ml |

Herstellungsverfahren:

Die Benzoesäureester werden in Äthanol gelöst und anschließend das Anisöl und das Methanol zugegeben. Dann wird die Wirksubstanz, Glycerin und Saccharin-Natrium im Wasser gelöst zugegeben. Die Lösung wird anschließend klar filtriert.

Patentansprüche

1. Imidazolderivate der allgemeinen Formel

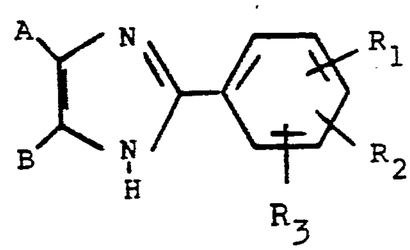

,(I)

in der

A und B zusammen mit den beiden dazwischenliegenden Kohlenstoffatomen eine Gruppe der Formel

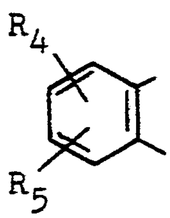 , 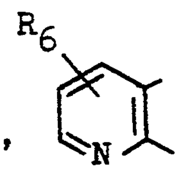 , 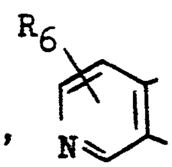 oder 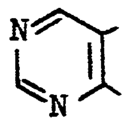 ,wobei

$R_4$ ein Wasserstoffatom- oder Halogenatom, eine Alkyl-, Hydroxy-, Alkoxy-, Trifluormethyl-, Cyano-, Carboxy-, Alkoxycarbonyl-, Aminocarbonyl-, Alkylaminocarbonyl-, Dialkylaminocarbonyl-, Hydroxyalkyl-, Nitro-, Amino-, Alkanoylamino-, Alkoxycarbonylamino-, Aminocarbonylamino-, Alkylaminocarbonylamino-, Dialkylaminocarbonylamino-, Alkansulfonylamino- oder N-Alkyl-alkansulfonylaminogruppe,

$R_5$ ein Wasserstoffatom- oder Halogenatom, eine Alkyl-, Hydroxy- oder Alkoxygruppe und

$R_6$ ein Wasserstoff- oder Halogenatom oder eine Alkylgruppe darstellen, wobei die vorstehend erwähnten Alkylteile jeweils 1 bis 3 Kohlenstoffatome enthalten können,

$R_1$ eine Alkansulfonyloxy-, Trifluormethansulfonyloxy-, Alkansulfonylamino-, N-Alkyl-alkansulfonylamino-, Trifluormethansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, wobei der Alkylteil der vorstehend genannten Gruppen jeweils 1 bis 3 Kohlenstoffatome enthalten kann, eine durch eine Amino-, Alkylamino-, Dialkylamino- oder cyclische Iminogruppe substituierte Sulfonylgruppe, wobei der Alkylteil jeweils 1 bis 5 Kohlenstoffatome und die Iminogruppe 4 bis 7 Kohlenstoffatome enthalten und gleichzeitig eine Methylengruppe in 4-Stellung der cyclischen Iminogruppe durch ein Schwefel- oder Sauerstoffatom ersetzt sein kann, eine Nitro- oder Cyangruppe,

$R_2$ eine Alkyl-, Alkoxy- oder Dialkylaminogruppe mit jeweils 1 bis 3 Kohlenstoffatomen in jedem Alkylteil und

$R_3$ ein Wasserstoffatom oder eine Alkoxygruppe mit 1 bis 3 Kohlenstoffatomen bedeuten, deren Tautomere und deren Säureadditionssalze.

2. Imidazolderivate der allgemeinen Formel I gemäß Anspruch 1, in der
A und B zusammen mit den beiden dazwischenliegenden Kohlenstoffatomen eine Gruppe der Formel

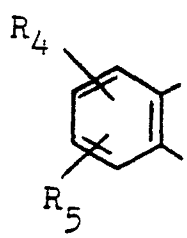 , 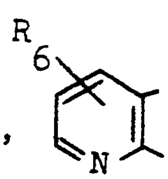 , 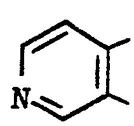 oder 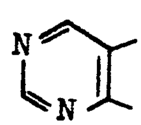 ,wobei

$R_4$ ein Wasserstoff-, Fluor-, Chlor- oder Bromatom, eine Trifluormethyl-, Cyan-, Methyl-, Hydroxy-, Methoxy-,

Hydroxymethyl-, Carboxy-, Methoxycarbonyl-, Aminocarbonyl-, Methylaminocarbonyl-, Nitro-, Amino-, Acetylamino-, Methoxycarbonylamino-, Methansulfonylamino-, Aminocarbonylamino- oder Methylaminocarbonylaminogruppe,

$R_5$ ein Wasserstoffatom, eine Methyl- oder Methoxygruppe und

$R_6$ eine Methylgruppe, ein Wasserstoff- oder Chloratom darstellen,

$R_1$ eine Alkansulfonyloxy-, Trifluormethansulfonyloxy-, Alkylsulfenylmethyl-, Alkylsulfinylmethyl-, Alkylsulfonylmethyl-, Alkansulfonylamino-, N-Alkyl-alkansulfonylamino-, Trifluormethansulfonylamino- oder N-Alkyl-trifluormethansulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1 bis 4 Kohlenstoffatomen,

$R_2$ eine Alkylgruppe mit 1 bis 3 Kohlenstoffatomen, eine Alkoxy- oder Dialkylaminogruppe mit 1 oder 2 Kohlenstoffatomen in jedem Alkylteil und

$R_3$ ein Wasserstoffatom oder die Methoxygruppe bedeuten, deren Tautomeren und deren Säureadditionssalze.

3. Imidazolderivate der allgemeinen Formel

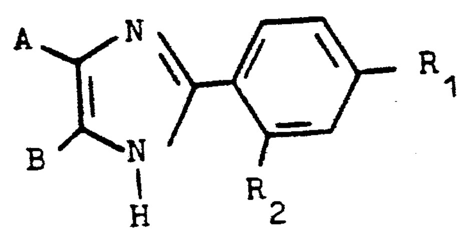

,(Ia)

in der

A und B zusammen mit den beiden dazwischenliegenden Kohlenstoffatomen eine Gruppe der Formel

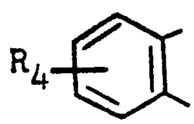 , 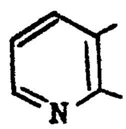 , 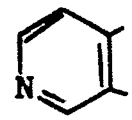 oder 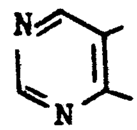 ,wobei

$R_4$ ein Wasserstoff- oder Fluoratom, eine Methyl-, Hydroxy-, Methoxycarbonyl-, Aminocarbonyl-, Amino-, Acetylamino- oder Cyangruppe darstellt,

$R_1$ die Methansulfonyloxy-, Trifluormethansulfonyloxy-, Methansulfonylamino-, Trifluormethansulfonylamino-, Methansulfonyl-methylamino-, Trifluormethansulfonyl-methylamino-, Methylsulfenylmethyl-, Methylsulfinylmethyl-, Methylsulfonylmethyl-, Cyan-, Aminocarbonyl-, Aminosulfonyl-, Methylaminosulfonyl- oder Dimethylaminosulfonylgruppe und
$R_2$ die Methoxy- oder Dimethylaminogruppe bedeuten, deren Tautomeren und deren Säureadditionssalze.

4. Imidazolderivate der allgemeinen Formel Ia gemäß Anspruch 3, in der

A und B zusammen mit den beiden dazwischenliegenden Kohlenstoffatomen eine Gruppe der Formel

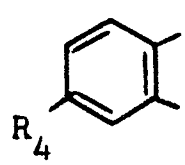 , 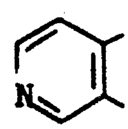 oder 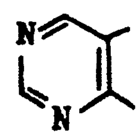 ,wobei

$R_4$ ein Wasserstoffatom, eine Hydroxy-, eine Cyan-, Aminocarbonyl- oder Acetylaminogruppe darstellt, $R_1$ die Methansulfonyloxy-, Methansulfonylamino-, N-Methyl-methansulfonylamino- oder Trifluormethansulfonyloxygruppe und $R_2$ die Methoxygruppe bedeuten, deren Tautomeren und deren Säureadditionssalze.

5. Benzimidazolderivate der allgemeinen Formel Ia gemäß Anspruch 3, in der $R_1$ , $R_2$ und $R_4$ wie im Anspruch 4 definiert sind, deren Tautomere und deren Säureadditionssalze.

6. Imidazo[4,5-c]pyridine der allgemeinen Formel Ia gemäß Anspruch 3, in der $R_1$ und $R_2$ wie im Anspruch 4 definiert sind, deren Tautomere und deren Säureadditionssalze.

7. Purine der allgemeinen Formel Ia gemäß Anspruch 3, in der $R_1$ und $R_2$ wie im Anspruch 4 definiert sind, deren Tautomere und deren Säureadditionssalze.

8. 8-(2'-Methoxy-4'-methansulfonyloxy-phenyl)-purin, dessen Tautomeres und dessen Säureadditionssalze.

9. 5-Cyan-2-(2'-methoxy-4'-methansulfonyloxy-phenyl)-benzimidazol, dessen Tautomeres und dessen Säureadditionssalze.

10. 2-(2'-Methoxy-4'-methansulfonylamino-phenyl)-imidazo-[4,5-c]pyridin, dessen Tautomeres und dessen Säureadditionssalze.

11. 2-(2'-Methoxy-4'-methansulfonyloxy-phenyl)-imidazo-[4,5-c]pyridin, dessen Tautomeres und dessen Säureadditionssalze.

12. Physiologisch verträgliche Säureadditionssalze mit anorganischen oder organischen Säuren der Verbindungen gemäß den Ansprüchen 1 bis 11.

13. Arzneimittel, enthaltend eine Verbidung gemäß den Ansprüchen 1 bis 11 oder ein physiologisch verträgliches Säureadditionssalz hiervon neben einem oder mehreren inerten Trägerstoffen und/oder Verdünnungsmitteln.

14. Arzneimittel gemäß Anspruch 13 zur Behandlung der Herzinsuffizienz.

15. Verfahren zur Herstellung von Imidazolderivaten der allgemeinen Formel I gemäß den Ansprüchen 1 bis 11, von deren Tautomeren und von deren Säureadditionssalzen, insbesondere von deren physiologisch verträglichen Säureadditionssalzen mit anorganischen oder organischen Säuren, dadurch gekennzeichnet, daß

a) eine gegebenenfalls im Reaktionsgemisch hergestellte Verbindung der allgemeinen Formel

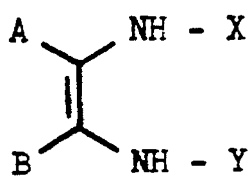

,(II)

in der
A und B wie im Anspruch 1 definiert sind,
einer der Reste X oder Y ein Wasserstoffatom und der andere der beiden Reste X oder Y oder beide Reste X und Y eine Gruppe der Formel

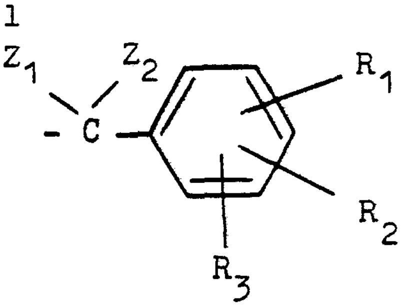

darstellen, in der

$R_1$ bis $R_3$ wie im Anspruch 1 definiert sind, $Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder $Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung von Verbindungen der allgemeinen Formel I, in der $R_1$ eine Alkylsulfinylmethyl- oder Alkylsulfonylmethylgruppe darstellt, eine Verbindung der allgemeinen Formel

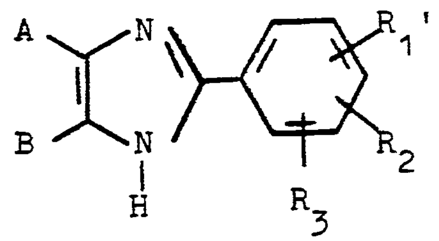

,(III)

in der A, B, $R_2$ und $R_3$ wie im Anspruch 1 definiert sind und $R_1'$ eine Alkylsulfenylmethyl- oder Alkylsufinylmethylgruppe mit jeweils 1 bis 3 Kohlenstoffatomen im Alkylteil darstellt, oxidiert wird oder

c) zur Herstellung von Verbindungen der allgemeinen Formel I, in der $R_1$ eine Alkansulfonyloxy-, Trifluormethansulfonyloxy-, Alkansulfonylamino-, N-Alkyl-alkansulfonylamino-, Trifluormethansulfonylamino- oder N-Alkyl-trifluormethansulfonylaminogruppe darstellt, eine Verbindung der allgemeinen Formel

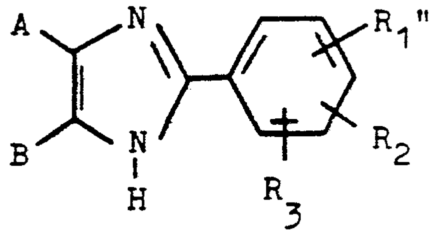

,(IV)

in der
A, B, $R_2$ und $R_3$ wie im Anspruch 1 definiert sind und $R_1$'' eine Hydroxy-, Amino- oder N-Alkylaminogruppe mit 1 bis 3 Kohlenstoffatomen im Alkylteil darstellt, mit einer Sulfonsäure der allgemeinen Formel

$$R_7 - SO_3H \qquad ,(V)$$

in der
$R_7$ eine Alkylgruppe mit 1 bis 3 Kohlenstoffatomen oder eine Trifluormethylgruppe darstellt, in Gegenwart eines wasserentziehenden und/oder die Säure oder das Amin aktivierende Mittel oder mit deren reaktionsfähigen Derivaten umgesetzt wird oder

d) zur Herstellung von Verbindungen der allgemeinen Formel I, in der $R_1$ eine durch eine Amino-, Alkylamino- oder Dialkylaminogruppe substituierte Carbonyl- oder Sulfonylgruppe darstellt, eine Verbindung der allgemeinen Formel

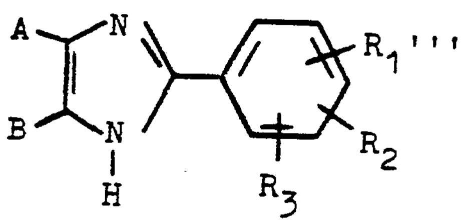

,(VI)

in der
A, B, $R_2$ und $R_3$ wie im Anspruch 1 definiert sind und $R_1$''' eine Carboxyl- oder Hydroxysulfonylgruppe darstellt, in Gegenwart eines wasserentziehenden oder die Säure aktivierenden Mittels oder eines reaktionsfähigen Derivates hiervon mit einem Amin der allgemeinen Formel

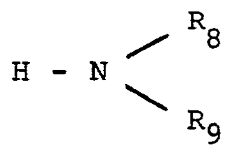

,(VII)

in der $R_8$ und $R_9$, die gleich oder verschieden sein können Wasserstoffatome oder Alkylgruppen mit 1 bis 5 Kohlenstoffatomen darstellen, oder mit einem reaktionsfähigen Derivat hiervon, falls $R_1'''$ die Carboxyl- oder Hydroxysulfonylgruppe darstellt, umgesetzt wird

und gewünschtenfalls anschließend eine so erhaltene Verbindung der allgemeinen Formel I, in der $R_1$ die Cyangruppe darstellt, mittels Alkoholyse und/oder Hydrolyse in eine entsprechende Verbindung der allgemeinen Formel I, in der $R_1$ eine Alkoxycarbonylgruppe mit insgesamt 2 bis 5 Kohlenstoffatomen oder die Carboxylgruppe darstellt, übergeführt wird und/oder

eine so erhaltene Verbindung der allgemeinen Formel I, in der $R_1$ und/oder $R_4$ die Cyangruppe darstellt, mittels Alkoholyse und/oder Hydrolyse in eine entsprechende Verbindung, in der $R_1$ eine Alkoxycarbonylgruppe mit insgesamt 2 bis 5 Kohlenstoffatomen, die Aminocarbonyl- oder Carboxylgruppe und/oder $R_4$ eine Alkoxycarbonylgruppe mit insgesamt 2 bis 4 Kohlenstoffatomen, die Aminocarbonyl- oder Carboxylgruppe darstellen, übergeführt wird und/oder

eine so erhaltene Verbindung der allgemeinen Formel I, in der $R_1$ und/oder $R_4$ die Carboxygruppe darstellt, mittels Veresterung in eine entsprechende Verbindung der allgemeinen Formel I, in der $R_1$ eine Alkoxycarbonylgruppe mit ins-

gesamt 2 bis 5 Kohlenstoffatomen und/oder $R_4$ eine Alkoxycarbonylgruppe mit insgesamt 2 bis 4 Kohlenstoffatomen darstellen, übergeführt wird und/oder

eine so erhaltene Verbindung der allgemeinen Formel I, in der $R_4$ eine Alkanoylaminogruppe darstellt, mittels Hydrolyse in eine entsprechende Verbindung der allgemeinen Formel I, in der $R_4$ eine Aminogruppe darstellt, übergeführt wird und/oder

eine so erhaltene Verbindung der allgemeinen Formel I, in der $R_4$ eine Nitrogruppe darstellt, mittels Reduktion in eine entsprechende Verbindung der allgemeinen Formel I, in der $R_4$ eine Aminogruppe darstellt, übergeführt wird und/oder

eine so erhaltene Verbindung der allgemeinen Formel I, in der $R_4$ eine Aminogruppe darstellt, mittels Überführung in ein Diazoniumsalz und anschließendem Erhitzen in eine entsprechende Verbindung der allgemeinen Formel I, in der $R_4$ die Hydroxygruppe darstellt, übergeführt wird und/oder

eine so erhaltene Verbindung der allgemeinen Formel I, in der $R_4$ eine Aminogruppe darstellt, mittels Carbamoylierung in eine entsprechende Verbindung der allgemeinen Formel I, in der $R_4$ eine Aminocarbonylamino- oder Alkylaminocarbonylaminogruppe darstellt, übergeführt wird und/oder

eine so erhaltene Verbindung der allgemeinen Formel I, in der $R_4$ eine Alkoxycarbonylgruppe darstellt, mittels Amidierung in eine entsprechende Verbindung der allgemeinen Formel I, in der $R_4$ eine Aminocarbonyl-, Alkylaminocarbonyl- oder Dialkylaminocarbonylgruppe darstellt, übergeführt wird und/oder

eine so erhaltene Verbindung der allgemeinen Formel I, in der $R_4$ eine Alkoxycarbonylgruppe darstellt, mittels Reduktion in eine entsprechende Verbindung der allgemeinen Formel I, in der $R_4$ eine Hydroxymethylgruppe darstellt, übergeführt wird und/oder

eine so erhaltene Verbindung der allgemeinen Formel I in ihre Säureadditionssalze, insbesondere in ihre physiologisch verträgliche Säureadditionssalze mit anorganischen oder organischen Säuren, übergeführt wird.

Patentansprüche (Benennungsland: AT)

1. Verfahren zur Herstellung von Imidazolderivaten der allgemeinen Formel

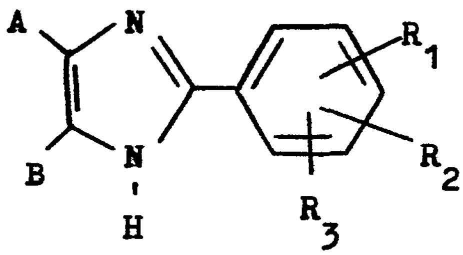

,(I)

in der

A und B zusammen mit den beiden dazwischenliegenden Kohlenstoffatomen eine Gruppe der Formel

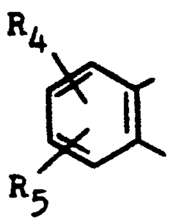 , 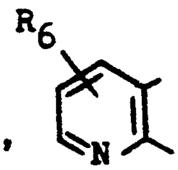 , 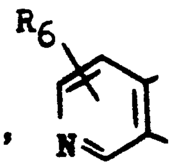 oder 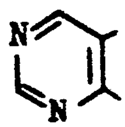 ,wobei

$R_4$ ein Wasserstoffatom- oder Halogenatom, eine Alkyl-, Hydroxy-, Alkoxy-, Trifluormethyl-, Cyano-, Carboxy-, Alkoxycarbonyl-, Aminocarbonyl-, Alkylaminocarbonyl-, Dialkylaminocarbonyl-, Hydroxyalkyl-, Nitro-, Amino-, Alkanoylamino-, Alkoxycarbonylamino-, Aminocarbonylamino-, Alkylaminocarbonylamino-, Dialkylaminocarbonylamino-, Alkansulfonylamino- oder N-Alkyl-alkansulfonylaminogruppe,

$R_5$ ein Wasserstoffatom- oder Halogenatom, eine Alkyl-, Hydroxy- oder Alkoxygruppe und

$R_6$ ein Wasserstoff- oder Halogenatom oder eine Alkylgruppe darstellen, wobei die vorstehend erwähnten Alkylteile jeweils 1 bis 3 Kohlenstoffatome enthalten können,

$R_1$ eine Alkansulfonyloxy-, Trifluormethansulfonyloxy-, Alkansulfonylamino-, N-Alkyl-alkansulfonylamino-, Trifluormethansulfonylamino-, N-Alkyl-trifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, wobei der Alkylteil der vorstehend genannten Gruppen jeweils 1 bis 3 Kohlenstoffatome enthalten kann, eine durch eine Amino-, Alkylamino-, Dialkylamino- oder cyclische Iminogruppe substituierte Sulfonylgruppe, wobei der Alkylteil jeweils 1 bis 5 Kohlenstoffatome und die Iminogruppe 4 bis 7 Kohlenstoffatome enthalten und gleichzeitig eine Methylengruppe in 4-Stellung der cyclischen Iminogruppe durch ein Schwefel- oder Sauerstoffatom ersetzt sein kann, eine Nitro- oder Cyangruppe,

$R_2$ eine Alkyl-, Alkoxy- oder Dialkylaminogruppe mit jeweils 1 bis 3 Kohlenstoffatomen in jedem Alkylteil und

$R_3$ ein Wasserstoffatom oder eine Alkoxygruppe mit 1 bis 3 Kohlenstoffatomen bedeuten, von deren Tautomeren und von deren Säureadditionssalzen, insbesondere von deren physiologisch verträglichen Säureadditionssalzen mit anorganischen oder organischen Säuren, dadurch gekennzeichnet, daß

a) eine gegebenenfalls im Reaktionsgemisch hergestellte Verbindung der allgemeinen Formel

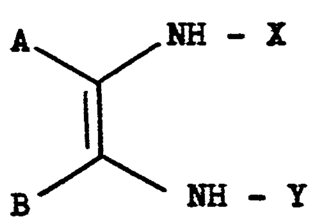

,(II)

in der
A und B wie eingangs definiert sind,

einer der Reste X oder Y ein Wasserstoffatom und der andere der beiden Reste X oder Y oder beide Reste X und Y eine Gruppe der Formel

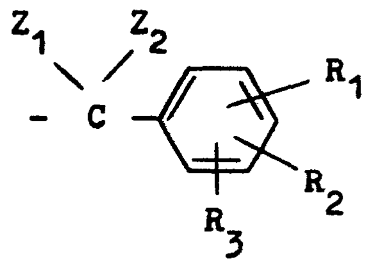

darstellen, in der

$R_1$ bis $R_3$ wie eingangs definiert sind,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder $Z_1$ und $Z_2$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung von Verbindungen der allgemeinen Formel I, in der $R_1$ eine Alkylsulfinylmethyl- oder Alkylsulfonylmethylgruppe darstellt, eine Verbindung der allgemeinen Formel

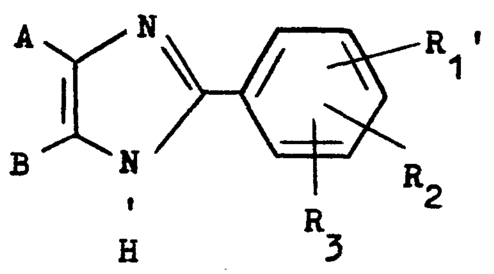

,(III)

in der
A, B, $R_2$ und $R_3$ wie eingangs definiert sind und
$R_1$' eine Alkylsulfenylmethyl- oder Alkylsufinylmethylgruppe mit jeweils 1 bis 3 Kohlenstoffatomen im Alkylteil darstellt, oxidiert wird oder

c) zur Herstellung von Verbindungen der allgemeinen Formel I, in der $R_1$ eine Alkansulfonyloxy-, Trifluormethansulfonyloxy-, Alkansulfonylamino-, N-Alkyl-alkansulfonylamino-, Trifluormethansulfonylamino- oder N-Alkyl-trifluormethansulfonylaminogruppe darstellt, eine Verbindung der allgemeinen Formel

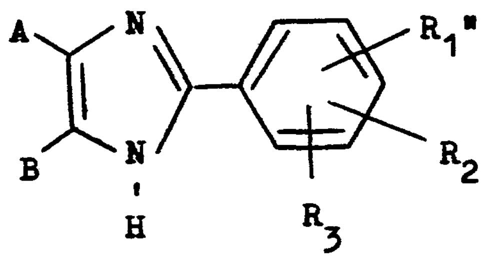

,(IV)

in der
A, B, $R_2$ und $R_3$ wie eingangs definiert sind und
$R_1$'' eine Hydroxy-, Amino- oder N-Alkylaminogruppe mit 1 bis 3 Kohlenstoffatomen im Alkylteil darstellt, mit einer Sulfonsäure der allgemeinen Formel

$$R_7 - SO_3H \quad ,(V)$$

in der
$R_7$ eine Alkylgruppe mit 1 bis 3 Kohlenstoffatomen oder eine Trifluormethylgruppe darstellt, in Gegenwart eines wasserentziehenden und/oder die Säure oder das Amin aktivierende Mittel oder mit deren reaktionsfähigen Derivaten umgesetzt wird oder

d) zur Herstellung von Verbindungen der allgemeinen Formel I, in der $R_1$ eine durch eine Amino-, Alkylamino- oder Dialkylaminogruppe substituierte Carbonyl- oder Sulfonylgruppe darstellt, eine Verbindung der allgemeinen Formel

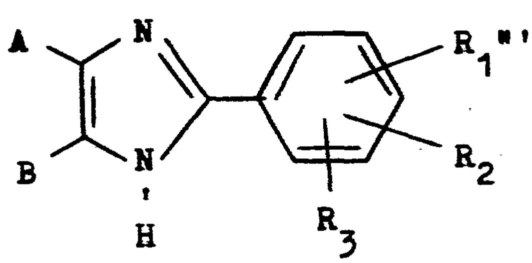

,(VI)

in der
A, B, $R_2$ und $R_3$ wie eingangs definiert sind und
$R_1'''$ eine Carboxyl- oder Hydroxysulfonylgruppe darstellt, in Gegenwart eines wasserentziehenden oder die Säure aktivierenden Mittels oder eines reaktionsfähigen Derivates hiervon mit einem Amin der allgemeinen Formel

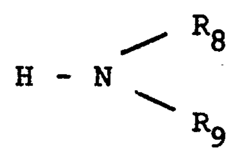

,(VII)

in der
$R_8$ und $R_9$, die gleich oder verschieden sein können Wasserstoffatome oder Alkylgruppen mit 1 bis 5 Kohlenstoffatomen darstellen, oder mit einem reaktionsfähigen Derivat hiervon, falls $R_1'''$ die Carboxyl- oder Hydroxysulfonylgruppe darstellt, umgesetzt wird

und gewünschtenfalls anschließend eine so erhaltene Verbindung der allgemeinen Formel I, in der $R_1$ die Cyangruppe darstellt, mittels Alkoholyse und/oder Hydrolyse in eine entsprechende Verbindung der allgemeinen Formel I, in der R eine Alkoxycarbonylgruppe mit insgesamt 2 bis 5 Kohlenstoffatomen oder die Carboxylgruppe darstellt, übergeführt wird und/oder

eine so erhaltene Verbindung der allgemeinen Formel I, in der $R_1$ und/oder $R_4$ die Cyangruppe darstellt, mittels Alkoholyse und/oder Hydrolyse in eine entsprechende Verbindung, in der $R_1$ eine Alkoxycarbonylgruppe mit insgesamt 2 bis 5 Kohlenstoffatomen, die Aminocarbonyl- oder Carboxylgruppe und/oder $R_4$ eine Alkoxycarbonylgruppe mit insgesamt 2 bis 4 Kohlenstoffatomen, die Aminocarbonyl- oder Carboxylgruppe darstellen, übergeführt wird und/oder

eine so erhaltene Verbindung der allgemeinen Formel I, in der $R_1$ und/oder $R_4$ die Carboxygruppe darstellt, mittels Veresterung in eine entsprechende Verbindung der allgemeinen Formel I, in der $R_1$ eine Alkoxycarbonylgruppe mit insgesamt 2 bis 5 Kohlenstoffatomen und/oder $R_4$ eine Alkoxycarbonylgruppe mit insgesamt 2 bis 4 Kohlenstoffatomen darstellen, übergeführt wird und/oder

eine so erhaltene Verbindung der allgemeinen Formel I, in der $R_4$ eine Alkanoylaminogruppe darstellt, mittels Hydrolyse in eine entsprechende Verbindung der allgemeinen Formel I, in der $R_4$ eine Aminogruppe darstellt, übergeführt wird und/oder

eine so erhaltene Verbindung der allgemeinen Formel I, in der $R_4$ eine Nitrogruppe darstellt, mittels Reduktion in eine entsprechende Verbindung der allgemeinen Formel I, in der $R_4$ eine Aminogruppe darstellt, übergeführt wird und/oder

eine so erhaltene Verbindung der allgemeinen Formel I, in der $R_4$ eine Aminogruppe darstellt, mittels Überführung in ein Diazoniumsalz und anschließendem Erhitzen in eine entsprechende Verbindung der allgemeinen Formel I, in der $R_4$ die Hydroxygruppe darstellt, übergeführt wird und/oder

eine so erhaltene Verbindung der allgemeinen Formel I, in der $R_4$ eine Aminogruppe darstellt, mittels Carbamoylierung in eine entsprechende Verbindung der allgemeinen Formel I, in der $R_4$ eine Aminocarbonylamino- oder Alkylaminocarbonylaminogruppe darstellt, übergeführt wird und/oder

eine so erhaltene Verbindung der allgemeinen Formel I, in der $R_4$ eine Alkoxycarbonylgruppe darstellt, mittels Amidierung in eine entsprechende Verbindung der allgemeinen Formel I, in der $R_4$ eine Aminocarbonyl-, Alkylaminocarbonyl- oder Dialkylaminocarbonylgruppe darstellt, übergeführt wird und/oder

eine so erhaltene Verbindung der allgemeinen Formel I, in der $R_4$ eine Alkoxycarbonylgruppe darstellt, mittels Reduktion in eine entsprechende Verbindung der allgemeinen Formel I, in der $R_4$ eine Hydroxymethylgruppe darstellt, übergeführt wird und/oder

eine so erhaltene Verbindung der allgemeinen Formel I in ihre Säureadditionssalze, insbesondere in ihre physiologisch verträgliche Säureadditionssalze mit anorganischen oder organischen Säuren, übergeführt wird.

2. Verfahren nach Anspruch 1 zur Herstellung von Imidazolderivaten der allgemeinen Formel

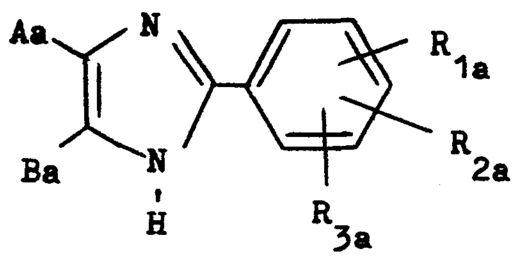

,(I')

in der

Aa und Ba zusammen mit den beiden dazwischenliegenden Kohlenstoffatomen eine Gruppe der Formel

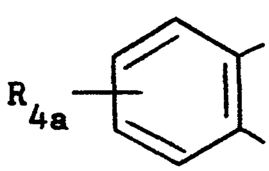

,

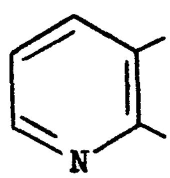

oder

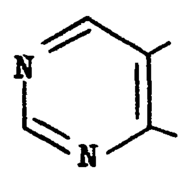

,wobei

$R_{4a}$ ein Wasserstoff- oder Halogenatom, eine Trifluormethyl-, Cyan- oder Alkoxygruppe mit 1 bis 3 Kohlenstoffatomen darstellt,

$R_{1a}$ eine Alkansulfonyloxy-, Alkansulfonylamino-, N-Alkylalkansulfonylamino-, Alkylsulfenylmethyl-, Alkylsulfinylmethyl- oder Alkylsulfonylmethylgruppe, wobei der Alkylteil

jeweils 1 bis 3 Kohlenstoffatome enthalten kann, eine durch eine Hydroxy-, Alkoxy-, Amino-, Alkylamino- oder Dialkylaminogruppe substituierte Carbonylgruppe, wobei der Alkylteil jeweils 1 bis 3 Kohlenstoffatome enthalten kann, eine durch eine Amino-, Alkylamino-, Dialkylamino- oder cyclische Iminogruppe substituierte Sulfonylgruppe, wobei der Alkylteil jeweils 1 bis 5 Kohlenstoffatome und die Iminogruppe 4 bis 7 Kohlenstoffatome enthalten und gleichzeitig eine Methylengruppe in 4-Stellung des Iminoringes durch ein Schwefel- oder Sauerstoffatom ersetzt sein kann, eine Nitro- oder Cyangruppe,

$R_{2a}$ eine Alkoxy- oder Dialkylaminogruppe mit jeweils 1 bis 3 Kohlenstoffatomen in jedem Alkylteil und

$R_{3a}$ ein Wasserstoffatom oder eine Alkoxygruppe mit 1 bis 3 Kohlenstoffatomen bedeuten, von deren

Tautomeren und von deren Säureadditionssalzen, insbesondere von deren physiologisch verträglichen Säureadditionssalzen mit anorganischen oder organischen Säuren, dadurch gekennzeichnet, daß

a) eine gegebenenfalls im Reaktionsgemisch hergestellte Verbindung der allgemeinen Formel

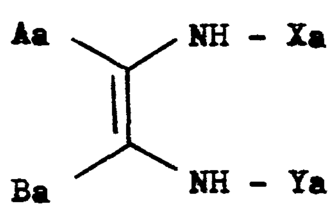

,(IIa)

in der

$A_a$ und $B_a$ wie eingangs definiert sind,
einer der Reste Xa oder Ya ein Wasserstoffatom und der andere der Reste Xa oder Ya oder beide Reste Xa und Ya eine Gruppe der Formel

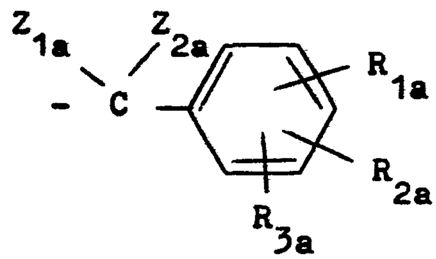

darstellen, in der

$R_{1a}$ bis $R_{3a}$ wie eingangs definiert sind,
$Z_{1a}$ und $Z_{2a}$, die gleich oder verschieden sein können, gegebenenfalls substituierte Aminogruppen oder gegebenenfalls durch niedere Alkylgruppen substituierte Hydroxy- oder Mercaptogruppen oder $Z_{1a}$ und $Z_{2a}$ zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung von Verbindungen der allgemeinen Formel Ia, in der $R_{1a}$ eine Alkylsulfinylmethyl- oder Alkylsulfonylmethylgruppe darstellt, eine Verbindung der allgemeinen Formel

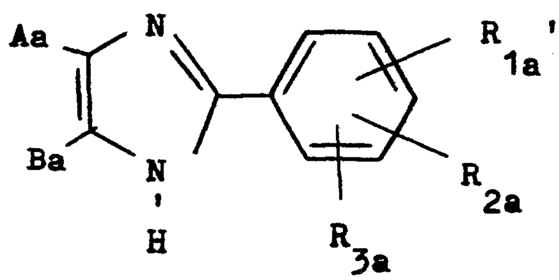

,(IIIa)

in der
Aa, Ba, $R_{2a}$ und $R_{3a}$ wie eingangs definiert sind und
$R_{1a}$', eine Alkylsulfenylmethyl- oder Alkylsulfinylmethylgruppe mit jeweils 1 bis 3 Kohlenstoffatomen im Alkylteil darstellt, oxidiert wird oder

0098448

c) zur Herstellung von Verbindungen der allgemeinen Formel Ia, in der $R_{1a}$ eine Alkansulfonyloxy-, Alkansulfonyl-amino- oder N-Alkyl-alkansulfonylaminogruppe darstellt, eine Verbindung der allgemeinen Formel

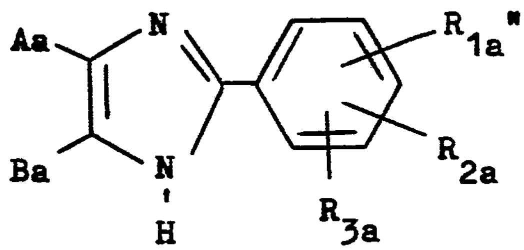

,(IVa)

in der

Aa, Ba, $R_{2a}$ und $R_{3a}$ wie eingangs definiert sind und $R_{1a}''$ eine Hydroxy-, Amino- oder N-Alkylaminogruppe mit 1 bis 3 Kohlenstoffatomen im Alkylteil darstellt, mit einer Sulfonsäure der allgemeinen Formel

$$R_{7a} - SO_3H \qquad ,(Va)$$

in der

$R_{7a}$ eine Alkylgruppe mit 1 bis 3 Kohlenstoffatomen darstellt, in Gegenwart eines wasserentziehenden und/oder die Säure oder das Amin aktivierende Mittel oder mit deren reaktionsfähigen Derivaten umgesetzt wird oder

d) zur Herstellung von Verbindungen der allgemeinen Formel Ia, in der $R_{1a}$ eine durch eine Amino-, Alkylamino- oder Dialkylaminogruppe substituierte Carbonyl- oder Sulfonylgruppe darstellt, eine Verbindung der allgemeinen Formel

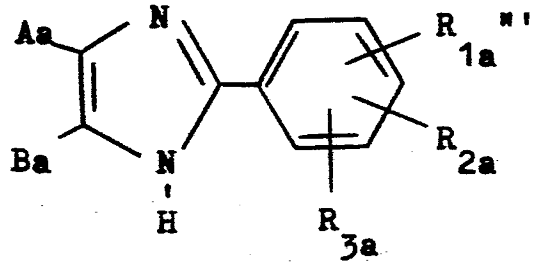

,(VIa)

in der

Aa, Ba, $R_{2a}$ und $R_{3a}$ wie eingangs definiert sind und

$R_{1a}$"' eine Carboxyl- oder Hydroxysulfonylgruppe darstellt, in Gegenwart eines wasserentziehenden oder die Säure aktivierenden Mittels oder eines reaktionsfähigen Derivates hiervon mit einem Amin der allgemeinen Formel

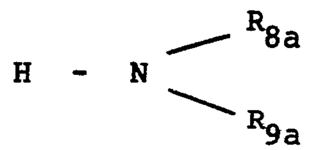

,(VIIa)

in der

$R_{8a}$ und $R_{9a}$, die gleich oder verschieden sein können, Wasserstoffatome oder Alkylgruppen mit 1 bis 5 Kohlenstoffatomen darstellen, oder mit einem reaktionsfähigen Derivat hiervon, falls $R_{1a}$"' die Carboxyl- oder Hydroxysulfonylgruppe darstellt, umgesetzt wird und gewünschtenfalls anschließend eine so erhaltene Verbindung der allgemeinen Formel Ia, in der $R_{1a}$ die Cyangruppe darstellt, mittels Alkoholyse und/oder Hydrolyse in eine entsprechende Verbindung der allgemeinen Formel Ia, in der $R_{1a}$ eine Alkoxycarbonylgruppe mit insgesamt 2 bis 5 Kohlenstoffatomen oder die Carboxylgruppe darstellt, übergeführt wird oder

eine so erhaltene Verbindung der allgemeinen Formel Ia, in der $R_{1a}$ die Carboxygruppe darstellt, mittels Veresterung in einer entsprechenden Verbindung der allgemeinen Formel Ia, in der $R_{1a}$ eine Alkoxycarbonylgruppe mit insgesamt 2 bis 5 Kohlenstoffatomen darstellt, übergeführt wird oder

eine so erhaltene Verbindung der allgemeinen Formel Ia in ihre Säureadditionssalze, insbesondere in ihre physiologisch verträgliche Säureadditionssalze mit anorganischen oder organischen Säuren, übergeführt wird.

Priorität: 01. Juli 1982 (P 32 24 512.2)

3. Verfahren nach den Ansprüchen 1 und 2, dadurch gekennzeichnet, daß die Umsetzung in einem Lösungsmittel durchgeführt wird.

4. Verfahren nach den Ansprüchen 1a, 2a und 3, dadurch gekennzeichnet, daß die Umsetzung bei Temperaturen zwischen 0 und 250°C durchgeführt wird.

5. Verfahren nach den Ansprüchen 1a, 2a, 3 und 4, dadurch gekennzeichnet, daß die Cyclisierung bei der Siedetemperatur des Reaktionsgemisches durchgeführt wird.

6. Verfahren nach den Ansprüchen 1a, 2a und 3 bis 5, dadurch gekennzeichnet, daß die Cyclisierung in Gegenwart eines Kondensationsmittels oder einer Base durchgeführt wird.

7. Verfahren nach den Ansprüchen 1b, 2b und 3, dadurch gekennzeichnet, daß die Umsetzung bei Temperaturen zwischen -80 und 100°C durchgeführt wird.

8. Verfahren nach den Ansprüchen 1c, 2c und 3, dadurch gekennzeichnet, daß die Umsetzung bei Temperaturen zwischen 0 und 100°C durchgeführt wird.

9. Verfahren nach den Ansprüchen 1d, 2d und 3, dadurch gekennzeichnet, daß die Umsetzung bei Temperaturen zwischen -25 und 250°C durchgeführt wird.